# EUROPEAN PATENT SPECIFICATION

(11) **EP 0 554 355 B1**
(45) Date of publication and mention of the grant of the patent: **07.01.1998**
(21) Application number: 91919934.9
(22) Date of filing: 21.10.1991
(51) Int. Cl.: C12Q 1/68, C12Q 1/02, C12Q 1/04, C12N 1/06, G01N 33/48, G01N 33/566, G01N 33/543

(54) **MEHODS AND PHARMACEUTICAL KITS USEFUL FOR DETECTING MICROORGANISMS ASSOCIATED WITH VAGINAL INFECTIONS**
VERFAHREN UND PHARMAZEUTISCHE SÄTZE ZUM AUFSPÜREN VON MIKROORGANISMEN ASSOZIIERT MIT VAGINALINFEKTIONEN
PROCEDES ET KITS PHARMACEUTIQUES UTILES POUR LA DETECTION DE MICROORGANISMES ASSOCIES A DES INFECTIONS VAGINALES

(30) Priority: 19.10.1990 US 600334
(43) Date of publication of application: 11.08.1993
(73) Proprietor: MICROPROBE CORPORATION, Bothell, WA 98021 (US)
(72) Inventor: SHEINESS, Diana, K., Bothell, WA 98021 (US); ADAMS, Trevor, H., Great Missenden Buckinghamshire HP169DD (GB)
(74) Representative: Thomson, Paul Anthony
(86) International application number: US9107763
(87) International publication number: WO9207096

(56) References cited:
- EP-A- 0 200 381
- EP-A- 0 272 009
- EP-A- 0 288 737
- EP-A- 0 335 633
- WO-A-90/01564
- US-A- 4 652 517
- West Indian Medical Journal, Vol. 38, No. 3, issued September 1989, P.N. LEVETT, "Bacterial Vaginosis", pages 126-132, see Abstract.

## Description

### Technical Field

The present invention relates to methods for diagnosing Bacterial Vaginosis that may be used in a medical practitioner's private office or in a more structured clinical environment, such as a hospital, a commercial clinical microbiology laboratory or the like. In addition, methods for simultaneously detecting Gardnerella vaginalis, Candida spp. and Trichomonas vaginalis in a biological sample are provided.

### Background of the Invention

Bacterial vaginosis (BV) is characterized by an alteration of the microbiological flora of a woman's vaginal area. This alteration involves a reduction in the normally present Lactobacilli cell number and an overgrowth of anaerobes and other microorganisms, including Gardnerella vaginalis (Gv).

During investigative attempts to understand this condition, the name of the disease has undergone an evolution. During the first quarter of the twentieth century, it was termed "leukorrhea." Later, doctors began referring to the disease as "non-specific vaginitis" (NSV). The sporadic use of the term NSV still persists. For a few years after the publication of a study by Gardner and Dukes in 1955, G. vaginalis was believed to be the sole causative agent of BV, and the disease was therefore known as "Gardnerella vaginalis vaginosis." Subsequent studies quickly established that G. vaginalis could be detected by microbiological culture in 10-50% of normal women, however. A few stubborn authors persist in the belief that G. vaginalis is the sole causative agent of BV and continue to use the aforementioned term appropriate to that belief, despite the publication of copious evidence that the presence of G. vaginalis does not necessarily indicate a disease state. In the 1980's, the term "bacterial vaginosis" entered the literature, and this term has become specifically associated with the clinical criteria that are presently relied upon to diagnose the disease. BV is not known to be caused by a single organism, and cannot therefore be diagnosed with microbiological culture being the sole criterion. Moreover, the recommended clinical criteria for diagnosis of BV do not include culture.

The clinical spectrum of BV varies from asymptomatic to obvious malodor and profuse discharge. The morbidity of BV has until recently been measured in terms of the high prevalence of the condition and the associated psychological distress, rather than in terms of the physical morbidity thereof. The increased intravaginal concentrations of bacteria and the shift to more virulent flora as a result of BV have been shown, however, to predispose a BV-positive woman to several adverse outcomes of pregnancy, such as chorioamnionitis, amniotic fluid infection, and puerperal infectious morbidity. BV has also been associated with premature birth (i.e., birth three to five weeks prior to the due date), pelvic inflammatory disease, postpartum endometritis, bacteremia, salpingitis, and the like. Some of these associations (e.g., polymicrobial upper genital tract infections) remain speculative.

BV cannot be diagnosed accurately by physical examination alone or in conjunction with symptom description. The current "gold standard" method of diagnosing BV involves the examination of four criteria:
1) presence of clue cells (determined microscopically),
2) white or gray adherent homogeneous discharge,
3) vaginal fluid pH > 4.5, and
4) fishy amine odor when vaginal fluid is mixed with 10% potassium hydroxide (KOH).

These criteria are applied in different ways by different investigators to diagnose BV. In one approach, the presence of clue cells plus two of the other three indicators are required. A BV patient diagnosed under an alternative approach must exhibit three of the four indicators.

Identification of clue cells requires special skills, since such cells are difficult to distinguish from other microscopically observable entities. Clue cells are vaginal epithelial cells having a large number of bacteria adhering thereto, such that the boundaries of the clue cells are blurred when viewed under a microscope. Recent studies suggest that bacteria, such as G. vaginalis, and anaerobic species, including Mobiluncus sp., adhere to vaginal epithelial cells, thereby forming clue cells.

An elevation in vaginal pH can occur for a variety of reasons. For example, infection with Trichomonas vaginalis or cervicitis, as well as BV, may cause a vaginal pH increase. Since pH is highly dependent on a number of variables, it is a sensitive parameter that exhibits poor specificity for BV. Use of pH alone to diagnose BV could result in an incidence of false positives as high as 47%.

In practice, physicians do not typically conduct pH and amine odor tests in their private offices. Furthermore, physicians rarely possess the skills necessary to adequately identify clue cells. As a result, the gold standard test is not often employed in the private physician office setting.

Another method for diagnosing BV that is used primarily in research protocols is the Gram stain. This diagnosis is difficult to perform, thereby rendering it unsuitable for use in a physician's office. Accurate reading of Gram stains requires special training, because the Gram stain analysis involves observation of both clue cells and the shift in vaginal flora described above. Moreover, this technique is less sensitive and less specific for BV than the gold standard method.

Currently, some physicians make use of a wet mount in conjunction with office vaginal examinations. A slide prepared from the patient's vaginal fluid is visually examined by the physician. When a BV-positive patient is examined by a physician practiced in making these difficult observations, such a slide will reveal an absence of the usual levels of Lactobacilli, which are large rods, and the presence of a large number of small rod-shaped bacteria, including Gardnerella vaginalis (Gv), Bacteroides, and Mobiluncus sp. The former two bacteria have straight rod shapes, while the latter bacterium exhibits a curved rod shape. Some physicians believe that clue cells may be identified through wet mount analysis, but such means of identification is not generally accepted as appropriate.

When first isolated, G. vaginalis was termed Haemophilus vaginalis. Later, G. vaginalis was reclassified as Corynebacterium vaginalis. Finally, G. vaginalis was placed into a new genus, Gardnerella, as it did not properly belong in either of the first two classifications.

Some investigators have attempted to determine whether the amount of G. vaginalis present in a sample is indicative of BV. They concluded that BV-positive women, on the average, have higher levels of G. vaginalis than BV-negative women. Considerable overlap was found to exist in the levels of G. vaginalis found in BV-positive and BV-negative women, however, thereby rendering the G. vaginalis cell level inconclusive evidence of the disease state. See Amsel et al., Am. J. Med. 74:14-22, 1983 and Eschenbach et al., Am. J. Obstet. Gynecol. 158:819-28, 1988.

If the BV-positive woman is not pregnant, metronidazole is most often prescribed to treat BV. An advantage of employing metronidazole treatment is that the medicament is active against Trichomonas vaginalis also. If the BV-positive woman is pregnant, metronidazole may be prescribed if the patient is beyond the first trimester. Alternatively, clindamycin may be prescribed at any time during pregnancy.

BV is one common cause of vaginal complaints. Other microorganisms commonly associated with such symptoms are Candida spp. and Trichomonas vaginalis. The organisms are typically identified by wet mount or by culture.

Oligonucleotide probes for use in hydridisation assays for the identification of Candida spp. are known in EP-A-0 335 633.

WO-A-90/01564 relates to nucleic acid hybridisation assays which allow for the detection of multiple target nucleic acids on a simple dipstick.

### Summary of the Invention

The present invention provides methods for diagnosing BV that are fast, accurate, and do not require an individual skilled in identifying clue cells, evaluating wet mounts or the like to assess the results. The methods of the present invention include: determining the pH of a vaginal sample; and determining the approximate number of G. vaginalis cells contained in the sample, wherein a pH value greater than about 4.5 and a number of G. vaginalis cells greater than or equal to a critical G. vaginalis cell number, indicates that the patient is BV-positive. These methods may be done manually or incorporate automated steps.

The methods of the present invention may also employ solid phase assay techniques to determine the concentration of G. vaginalis cells in a sample, wherein an elevated level of G. vaginalis cells in combination with a vaginal pH above about 4.5 indicate a BV-positive state. Such a procedure is preferably accomplished within about 6 hours or less.

The present invention also provides pharmaceutical kits for use in the methods of the present invention. Such pharmaceutical kits include: a first indicator capable of indicating pH; and a second indicator capable of indicating G. vaginalis cell level. These indicators of the kits of the present invention may be structurally integrated. In addition, the G. vaginalis cell level indicator of the kits of the present invention may be capable of binding to or have bound thereto either an oligonucleotide sequence that is complementary to G. vaginalis nucleic acid or an antibody that is specific for G. vaginalis.

The preferred G. vaginalis cell number indicator is a diagnostic dipstick. Such a G. vaginalis indicator preferably includes a bead to which the oligonucleotide or antibody is capable of binding or is bound.

In a second embodiment of the present invention, methods for simultaneously detecting Gardnerella vaginalis, Candida spp. and Trichomonas vaginalis in a biological sample are described. Such methods include exposing prokaryotic and eukaryotic cells that may be present in the biological sample to a lysis solution. This solution, alone or in combination with a first hybridization solution, releases target nucleic acid. The sample in lysis solution is combined with a first hybridization solution and a dipstick having capture oligonucleotide-coated beads attached thereto. The dipstick contains at least three beads capable of specifically hybridizing with Gardnerella vaginalis, Candida spp., and Trichomonas vaginalis nucleic acid. Target nucleic acid captured on the beads is detected using signal oligonucleotides capable of specifically hybridizing with the three target microorganisms. Kits for simultaneous detection of Gardnerella vaginalis, Candida spp. and Trichomonas vaginalis are also provided. The kits contain one or more dipsticks capable of specifically capturing Gardnerella vaginalis, Candida spp. and Trichomonas vaginalis nucleic acid, and a container including at least two signal oligonucleotides capable of specifically hybridizing with the three target microorganisms.

### Detailed Description of the Invention

The present invention is directed to methods and kits useful in the diagnosis of BV. The methods of the present invention include the steps of determining the pH of a vaginal sample obtained from a patient and determining the approximate number of G. vaginalis cells contained in the sample. A patient sample having a pH value greater than 4.5, in combination with a G. vaginalis cell number greater than or equal to a critical G. vaginalis cell number, indicates that the patient is BV-positive.

In almost every instance, the vaginal pH in women with BV is higher than pH 4.5, whereas the vaginal pH of women who are clinically negative for BV is almost always less than 4.5. However, pH alone is not diagnostic for BV, because other conditions can lead to elevated vaginal pH. While women with BV have elevated levels of G. vaginalis in their vaginal tract, enumeration of G. vaginalis, taken alone, is not a good indicator of BV, because BV-negative women frequently have elevated levels of G. vaginalis in their vaginas. The present invention permits rapid measurement of pH and G. vaginalis levels in a patient sample, as an indication of Bacterial Vaginosis.

The present invention makes use of the finding that a pH > 4.5 in combination with an elevated level of G. vaginalis in a vaginal sample are diagnostic for BV, even though each parameter taken alone is not diagnostic. The relevant value for pH (i.e., pH > 4.5) is one part of the standard, recommended clinical criteria for diagnosis of BV. Prior studies of G. vaginalis in BV-positive women have primarily ascertained the presence or absence of the organism. A few studies used semi-quantitative microbiology to establish that the G. vaginalis cell numbers are generally higher in BV-positive women than in BV-negative women.

The claimed methods and kits involve determining whether the G. vaginalis cell number in a patient sample is greater than or equal to a critical G. vaginalis cell number. The critical G. vaginalis cell number is a predetermined cell number that is associated with BV. The critical G. vaginalis cell number may vary with the sample collection method employed, and is defined herein to be either cell number or cell concentration. To initially identify the critical G. vaginalis cell number corresponding to a particular sampling method and assay procedure, the sampling method will be used to obtain specimens from a sufficient number of women that have been clinically diagnosed as BV-positive or BV-negative to establish the critical G. vaginalis cell number. A range of G. vaginalis cell numbers from the BV-positive women will be determined, and the critical G. vaginalis cell number will represent the bottom of this range. This critical number is tied to the selected sampling method. Determination of the critical G. vaginalis cell number will be dependent upon the sample collection procedure, such as vaginal wash, vaginal swab, or other sample obtaining means, as well as the assay used to measure the number of G. vaginalis cells present in that sample within a period of 6 hours or less. The G. vaginalis cell number may be measured by nucleic acid hybridization techniques, by techniques involving antibody-antigen interactions, or by any other method whose results can be correlated with a G. vaginalis cell number or G. vaginalis cell concentration. While the critical G. vaginalis cell number may be dependent on the methods chosen for sample collection and for G. vaginalis enumeration, the critical G. vaginalis cell number will be consistent over a 10-fold range for any selected protocol.

Using the selected sampling method and assay procedure, the G. vaginalis cell number of the sample will be established by comparing patient test results to a standard curve generated by concurrently evaluating standards derived from a known number of purified, cultured G. vaginalis. Serial dilutions of quantitated, cultured G. vaginalis are subjected to the same assay procedures as the patient samples. The signal intensity of each patient sample is compared to signal intensities of the diluted standards, and the patient sample can be correlated with an amount of G. vaginalis equivalent to that in the matching standard dilution.

Alternatively, a specific molecule that serves as the target of the G. vaginalis assay can be purified from a known number of cultured G. vaginalis and used as a standard for comparison to the patient sample results. For example, if 16S rRNA is the target of the assay, purified 16S rRNA could serve as the standard, with a given amount of 16S rRNA corresponding to a known number of G. vaginalis cells. In this instance, the patient sample results could be expressed in units equivalent to molecules of 16S rRNA; BV-positive and BV-negative women are characterized according to how many 16S rRNA equivalents are present in the sample. This approach could be used for any other purified molecule obtained from a known number of G. vaginalis cells. If desired, a separate experiment could determine the number of 16S rRNA molecules per G. vaginalis cell, and thereafter the 16S rRNA standard could be used, but the results could be expressed per G. vaginalis cell number. Since the number of target molecules (such as 16S rRNA) per cell can vary during different stages in the G. vaginalis cell growth cycle, the number of correlate molecules per cell should be established using a known number of G. vaginalis cells that are in the mid-log phase of growth. For consistency, this numerical equivalency will be determined only once, and thereafter the purified target molecule, such as 16S rRNA, can be used as a standard, with conversion to numbers of G. vaginalis cells according to the established numerical equivalency.

Once the selected method has been used to determine the number of G. vaginalis cells per sample fron the BV-positive and the BV-negative groups, the critical G. vaginalis cell number may be determined (i.e., the minimum number of G. vaginalis cells associated with BV-positive women). While some of the BV-negative women may have G. vaginalis cell numbers greater than the critical level, these women will nearly always be found to have a vaginal pH < 4.5, and thus will be diagnosed as BV-negative when the diagnostic kit instructions are followed.

Within the present invention, a critical cell number may be determined for an organism other than G. vaginalis in a manner similar to that set forth above for determining critical G. vaginalis cell number. For instance, the critical cell number for women presenting with Candidiasis may be determined relative to indigenous Candida levels found in the vagina.

The concentrations of the reaction components of a solid phase assay kit of the present invention will be adjusted to ensure that a positive signal will be obtained only if the amount of G. vaginalis present is equal to or greater than the critical G. vaginalis cell number. The critical G. vaginalis cell number will generally range from about 5 x 10⁶ to about 5 x 10⁹ cells per ml of vaginal fluid, with about 8 x 10⁶ to about 10⁹ cell/ml of vaginal fluid preferred, and about 2 x 10⁷ cells/ml of vaginal fluid particularly preferred. For the purposes of this description, "about 2 x 10⁷ cells/ml of vaginal fluid" means a G. vaginalis cell level within the range from about 5 x 10⁶ cells/ml to about 5 x 10⁹ cells/ml.

Vaginal fluid samples that may be tested in accordance with the present invention may be obtained in any conventional manner. Exemplary sample obtaining methodologies involve the use of a vaginal swab, vaginal wash techniques or the like.

The pH determining step of the methods of the invention may be accomplished by conventional techniques, such as contacting a sample, such as a vaginal swab or speculum, with pH paper or another pH indicating substrate and observing an alteration in the color of the paper or substrate that is indicative of relevant sample pH. The pH indicator may be included within a diagnostic kit of the present invention as a separate structural unit or as a portion of the structure of a diagnostic indicator card or dipstick. Alternatively, the pH indicator might be provided by the clinician. In addition, pH may be measured with electrodes or with commercially available pH indicators; alternatively, known pH indicators can be immobilized on a solid support.

Similarly, the G. vaginalis cell level may be determined by any method yielding an accurate measurement thereof that may be performed in 6 hours or less. Consequently, probes, including oligonucleotide sequences that are complementary to G. vaginalis DNA or RNA, antibodies selective for G. vaginalis or the like, may be used for this purpose. Direct hybridization assay techniques may be used in making the G. vaginalis cell level determination. Sandwich assay techniques employing oligonucleotide probes or antibodies are preferred.

In another embodiment of the present invention, several microorganisms associated with vaginal complaint are detected simultaneously and rapidly using one sample preparation. When patients present with vaginal complaints, microorganisms commonly associated with such symptomology include Candida spp. and Trichomonas vaginalis, as well as Gardnerella vaginalis. Therefore, a method to quickly, easily and simultaneously detect the presence of these three organisms in a single biological sample would be advantageous. The claimed invention describes lysis conditions suitable for releasing target nucleic acid from each of the microorganisms (representing both prokaryotic and eukaryotic cells). The released nucleic acids, and preferably released ribosomal RNAs, are then simultaneously processed and detected in a sandwich assay.

The present invention also contemplates diagnostic kits for determining whether a patient is afflicted with BV, including a first indicator capable of indicating pH; and a second indicator capable of indicating a G. vaginalis cell number or level associated with the disease state. Indicators of the presence of other organisms, such as Candida albicans, Trichomonas vaginalis, Neisseria gonorrhoeae, Chlamydia sp., Mobiluncus sp., Bacteroides sp., Mycoplasma sp. or the like, may also be included in the kits of the present invention. In this manner, the kits of the present invention may be employed to detect BV, vaginitis or cervicitis.

Specifically, the kit may contain a strip of pH indicating paper and a diagnostic indicator card, such as a dipstick. A cell disruption buffer/hybridization solution or a ligand incubation solution (optionally containing appropriate signal moieties) may also be included within the kits of the present invention to facilitate employment of sandwich assay techniques. Alternatively, the pH indicator may be structurally integrated with the G. vaginalis cell number indicator. The kits may be used in manual or semi-automatic testing procedures, and are preferably employable in sandwich assay techniques.

When simultaneously detecting the presence of more than one target nucleic acid, several parameters are considered. For instance, lysis solutions that effectively release nucleic acid from each target microorganism must be determined. Exemplary lysis solutions may include buffers, detergents, enzymes, reducing agents, organic solvents, antibiotics, chaotropic agents, cations, chelates, preservatives or combinations thereof. Preferred capture oligonucleotides are selected for both target specificity and duplex formation with target in a solid phase sandwich assay format. The latter characteristic is particularly important when the target is ribosomal RNA. Signal oligonucleotides may be selected from a range of specificities. That is, signal oligonucleotides may be specific for prokaryotic target nucleic acid or eukaryotic target nucleic acid, or may specifically hybridize with the genus and/or species of the target. The selection of capture oligonucleotides, signal oligonucleotides, lysis conditions, concentration of capture oligonucleotide on a bead, hybridization conditions, signal/detection systems and conditions, and combinations thereof may impact the sensitivity and selectivity of detection of a particular panel of microorganisms. However, one skilled in the art of nucleic acid sandwich hybridization assays can determine modifications or adjustments of these parameters that optimize simultaneous detection of a particular panel of target microorganisms.

In solid phase sandwich assays involving oligonucleotide probe technology (described generally by Dunn et al., Cell 12:23-36, 1977), the target nucleic acid is usually released from the target microorganism into a solution. The target nucleic acid solution is then transferred to or adjusted to solution conditions in which hybridization to a solid support can occur. Such an assay technique may also be employed on a portion of the original patient sample that has been manipulated in some manner (i.e., purified, treated chemically or the like).

In a preferred embodiment, target nucleic acid is typically sequestered (captured) from the original patient sample by hybridization (i.e., pairing of complementary bases) with capture oligonucleotide probes that are covalently immobilized on the surface of a solid support. The captured target nucleic acid is then hybridized to a signal oligonucleotide probe having a detectable label bound thereto, or having the capability of binding to a moiety having a detectable label bound thereto. The signal probe is specific for an alternative site on the target nucleic acid. Alternatively, signal hybridization can be performed simultaneously with capture hybridization by including the signal probe within, for example, the hybridization solution. This results in a "sandwich" of the capture oligonucleotide probe:target nucleic acid:signal oligonucleotide probe. The solid support is washed to remove unhybridized material, and the labeled nucleic acid is then measured in accordance with detectable characteristics of the label.

In sandwich assays involving antigen/antibody technology, antigen is either present in the original sample, extracted therefrom or released from organisms contained in the original sample by reagents that disrupt the cell wall and/or membrane. The antigen is sequestered (captured) from the test sample by interaction with antigen-specific antibody that is covalently immobilized on the surface of a solid support. The captured target antigen may then be incubated with a signal antibody having a detectable label bound thereto, or having the capability of binding to a moiety having a detectable label bound thereto. The signal antibody is specific for an alternative site on the target antigen. Alternatively, signal antibody binding can be performed simultaneously with capture by including the signal antibody within, for example, the incubation solution. This results in a "sandwich" of the capture antibody:target antigen:signal antibody. The solid support is washed to remove unbound signal antibody, and the labeled antigen is then measured in accordance with detectable characteristics of the label.

In the sandwich assays described above, biotin/avidin or biotin/streptavidin technology may also be employed. Specifically, the biotin/avidin interaction may be exploited to couple a more generalized detection system to the oligonucleotide probe or antigen/antibody sandwich assays. For example, the signal probe of the sandwich assay may be covalently bound to biotin. This biotin-labeled signal probe can then be incubated with avidin or streptavidin, its complementary ligand, having a detectable label bound thereto. This results in the detection of a "sandwich" of the capture oligonucleotide probe:target nucleic acid:signal oligonucleotide probe/biotin: avidin/detectable label. In this embodiment, avidin/ detectable label can be prepared in large scale and can bind to signal/biotin moieties in a variety of sandwich assays. Other ligand pairs are also useful in the solid phase assays of the present invention. Exemplary of such additional ligand pairs are lectin:sugar, hormone:hormone receptor and the like.

The preferred solid support for use in the diagnostic methods and kits of the present invention is a polymer-coated bead having activatable amine groups, as described in EP-A-0 455 905 and WO-A-91/08307. Bead solid supports useful in accordance with the present invention possess certain advantages over known membrane or bead solid supports, to which capture nucleic acid sequences are noncovalently bound. The capture rate of target nucleic acid sequences is improved 5- to 25-fold. Virtually all of the capture nucleic acid sequence bound to the solid support is available for hybridization with a complementary sequence. Also, the quantity of immobilized capture nucleic acid can be increased approximately 20-fold on an apparent surface area basis. The preferred solid supports may be more easily manufactured than their prior art counterparts. In addition, such beads possess covalently immobilized capture nucleic acid sequences (oligonucleotides) that can withstand denaturation temperatures in excess of 90°C for 10 or more minutes. Also, a multisite dipstick employing a plurality of bead solid supports can be constructed, leading to miniaturization of the dipstick device and reduction in the volume of sample required for detection. These advantages contribute to increased sensitivity when a sandwich assay hybridization format is used to diagnose BV in accordance with the present invention.

Covalent immobilization of an oligonucleotide on a solid support, preferably a bead, may be accomplished by the following procedure: treating a solid support with an activating agent, if necessary; reacting the treated solid support with an amine-containing polymer, whereby the polymer coats the solid support; activating an oligonucleotide with a monofunctional or multifunctional reagent, preferably the homotrifunctional reagent, cyanuric chloride; and conjugating the activated oligonucleotide and the polymer-coated solid support. The unreacted amines on the support surface are then acylated to impart the proper surface charge to the solid support surface, thereby minimizing non-specific interaction with nucleic acid.

For the purposes of this description, the term "nucleic acid" includes single stranded DNA and RNA, and double stranded DNA, RNA and DNA-RNA hybrids.

For the purposes of this description, the term "lysis" refers to rendering nucleic acids or antigens of cells contained in the test sample available to the capture probe or ligand. The cells acted upon by a lysing agent may or may not be structurally altered in a manner detectable by visual inspection under a microscope. Exemplary lysing solutions useful in practicing the present invention include detergents (e.g., anionic, cationic and/or non-ionic), chaotropic agents, enzymes (e.g., proteases, sialidases, glycosidases, glucanases, chitinases, lyticase, lysozyme, lipases, hyaluronidase, and the like), organic solvents, reducing agents (e.g., dithiothreitol, β-mercaptoethanol or the like), chelating agents (e.g., EDTA, EGTA and the like), antibiotics or the like, used alone or in combination. One preferred embodiment of the present invention involves pretreatment with one or more enzymes, organic solvent, or reducing agent prior to addition of a chaotropic agent. Lysis may be conducted at ambient or at elevated temperature.

For the purposes of this description, the term "solid support" refers to any surface that is transferable from solution to solution and that has means for attaching a bead. For instance, a bead may be attached to a perforation or depression in the solid support.

For the purposes of this description, the term "bead" refers to a structure for conducting oligonucleotide-based assays, and includes beads, microbeads, powder, membranes, microtiter wells, strings, fabric, plastic strips, films, or any surface onto which nucleic acid probes or antigen may be immobilized. Solid supports useful in the practice of the present invention may exhibit natural or intrinsic fluorescence. For instance, the term "bead" encompasses any type of solid or hollow sphere, ball, bearing, cylinder, or other similar configuration composed of plastic, ceramic, metal, nylon, or polymeric material onto which a nucleic acid or antigen can be covalently immobilized. As such, the term also includes nylon string. Preferably, spherical nylon beads are employed in the methods and kits of the present invention. A preferred diameter range for such beads is from about 0.01 inch to about 0.5 inch, more preferably from about 0.05 inch to about 0.1 inch, and most preferably about 0.06 inch (corresponding to commercially available 3/32 inch nylon beads). Additionally, it is preferred that the nylon beads are burnished or roughened before treating with an alkylating agent.

In a preferred embodiment of the present invention, a nylon bead (or beads or any composition or structure of nylon) is activated by treating the bead with an alkylating agent. Alkylating agents react with amides present in the nylon polymer to form reactive imidate esters. Preferred alkylating agents include, but are not limited to, dialkyl sulfates, alkyl triflates, alkyldiphenyl sulfonium salts, alkyl perchlorates, and, more preferably, trialkyloxonium salts. Exemplary trialkyloxonium salts useful in the present invention include lower alkyl salts, such as trimethyloxonium and triethyloxonium salts. Exemplary salt counterions are hexachloroantimonate, hexafluorophosphate, and tetrafluoroborate, with the last named counterion being preferred.

An alkylating agent solvent that does not dissolve nylon or render nylon tacky during the alkylation procedure is preferably employed in activating the preferred nylon solid support useful in the diagnostic methods and kits of the present invention. Non-nucleophilic organic solvents, such as dichloromethane, dimethylsulfoxide, tetrahydrofuran, and others, are exemplary solvents that may be employed for this purpose. N-methyl-pyrrolidone is preferred, because it is a solvent that supports alkylation.

The resulting bead surface imidate esters are then reacted under suitable conditions with an amine-containing polymer, whereby amidine residues are formed. Any primary or secondary amine-containing polymer can be employed to form amidine residues, thus covalently immobilizing the polymer onto the surface of the bead. Poly(ethylene-imine), polyallylamine, and polyvinylamine are preferred examples. The preferred solvent used to dissolve the polymer during the conjugation of the polymer to the activated nylon bead is N-methyl-pyrrolidone.

Alternatively, nylon can also be partially hydrolyzed to yield reactive amine or carboxyl groups (capable of subsequently reacting with amine- or carboxyl-containing polymers). In this manner, an activated solid support coated with reactive moieties may be produced.

Dipsticks are preferably employed in the methods and kits of the present invention. Dipsticks having utility in nucleic acid hybridizations indicating G. vaginalis, and including a nonporous bead support and a means for attaching a bead thereto, are so employed. Nonporous bead supports are known in the art. An example of bead attachment to a nonporous bead support involves a perforation or perforations (or a depression or depressions) in the nonporous bead support, wherein beads can be attached. Preferably, perforations are employed and the beads are attached by pressure fit within the circumference of the such perforations. One of ordinary skill in the art will appreciate that other bead attachment methods may be employed in production of a dipstick useful in practicing the present invention.

The dipstick used in the methods and kits of the present invention may contain more than one bead. Preferably the dipstick will contain from about two to one hundred beads, and more preferably about two to ten beads, each within its own perforation. More preferably, the plurality of bead-containing perforations will be situated in a row along one edge of the dipstick. Such a dipstick can function as an indicator card. Specifically, multiple beads with covalently attached capture oligonucleotide probes or antigens with different sequences or specificities are closely aligned on a multisite dipstick, thereby facilitating the detection of a multiplicity of organisms in a single biological sample. A particular bead may contain oligonucleotides representing a plurality of nonidentical nucleic acid sequences (for example, sequences from a group of related organisms), or a bead may only contain a plurality of identical oligonucleotides having a specific nucleic acid sequence.

Preferably, a dipstick useful in the practice of the present invention will include a bead specific for G. vaginalis, a positive control, a negative control and, optionally, a pH indicator bead or strip. Conventional pH indicators can be absorbed onto or covalently attached to a solid support to be incorporated into a solid phase assay format. See, for example, "Indicators," Bishop (ed.), in Belcher et al. (eds.), "International Series of Monographs in Analytical Chemistry," p. 51, Pergammon Press, 1972. A pH-indicating strip may also be included in the kit as a separate structural unit, however. Of course, the pH of the test sample will be determined prior to combination of the test sample with one or more solutions of the present invention.

In another embodiment, the dipstick may include beads specific for Trichomonas vaginalis and/or Candida spp., in addition to a bead specific for G. vaginalis, a procedural positive control, and a procedural negative control. Such a dipstick will indicate whether the sample cell number is greater than or equal to the critical cell number for these two non-BV-related vaginal pathogens, and will provide a more comprehensive diagnostic tool, since vaginitis may also be detected (and causative organisms distinguished) through the practice of this embodiment of the present invention. In another embodiment, the dipstick may include beads specific for Neisseria gonorrhoeae, Chlamydia sp., Mobiluncus sp., Bacteroides sp. and/or Mycoplasma sp., in addition to a bead specific for G. vaginalis, a procedural positive control, and a procedural negative control. Such a dipstick will indicate whether the sample cell number(s) is equal to or exceeds the critical cell number determined for an individual organism associated with cervicitis (Neisseria gonorrhoeae, Chlamydia sp.) or potentially pathogenic vaginal infection. Such dipsticks may be packaged with one or more lysis reagents capable of freeing target nucleic acid sequences for hybridization with the capture and signal probe components of the kits of the present invention.

The development of a dipstick-based assay capable of detecting the presence of multiple target nucleic acids requires identification of three key parameters: (1) the lysis or release of target nucleic acid from the sample; (2) the capture and detection of the target nucleic acid; and (3) the reagent components of the assay format. If the reagent components remain constant (i.e., hybridization conditions, wash solutions, detection enzyme, and the like), one skilled in the art can identify and select specific capture and signal oligonucleotides for hybridization with the target nucleic acid. When the conditions for capture and detection of the target nucleic acid are defined, one skilled in the art can determine lysis conditions that allow the release of the target nucleic acid from a biological sample. The lysis conditions may vary for each of the target microorganisms, and a balance of lytic conditions allows the simultaneous detection of the target organisms. If these parameters are identified, the critical cell number for each microorganism can be determined in biological samples. The critical cell number may vary with the method used to acquire the sample, and can be defined as the cell number that leads to symptomatic presentation of a disorder. Symptomatic presentation is particularly relevant for disorders where an asymptomatic presence of the microorganism is possible.

In a further embodiment of the claimed invention, oligonucleotide-coated beads may be employed in a microtiter well format. This format may be advantageously used when a large number of patient samples are assayed. Further, the microtiter well format is compatible with signal probes that are detected through means of a soluble reaction product.

Table 1 set forth below summarizes the results obtained when exemplary lysis reagents were incubated with the indicated organisms in the presence of patient samples. The first method, designated (1), involves direct immersion of the sample in 3 M guanidinium thiocyanate (GuSCN). The second method (2) requires heating to 65°C in 1 mg/ml proteinase K, followed by the addition of GuSCN to a final concentration of 3 M. The third method (3) requires heating at 85°C in a buffered solution containing detergents, followed by addition of GuSCN to a final concentration of 3 M.

**TABLE 1**

| LYSIS REAGENTS | | | |
|---|---|---|---|
| Organism | (1) | (2) | (3) |
| Candida | - | + | + |
| Candida, spiked^{a} | - | + | + |
| Gardnerella | -/+ | + | + |
| Gardnerella, spiked^{a} | - | + | + |
| Trichomonas | + | + | + |
| Trichomonas, spiked^{a} | + | - | + |

| | | | |
|---|---|---|---|
| ^{a} "Spiked" means a swab was taken from a BV-negative woman, and about 5 x 10⁷ cultured organisms were placed directly onto the swab. The swab is then processed as if it were a patient sample. | | | |

A plus sign indicates successful detection of target nucleic acid in the presence of vaginal fluid samples. Using these reagents, patient samples are collected into proteinase K followed by GuSCN addition (method (2)) for Candida albicans and G. vaginalis detection, and into 3 M GuSCN (method (1)) for Trichomonas vaginalis detection. Alternatively, two patient samples are collected simultaneously, or one patient sample would be divided into aliquots, and incubated in 5 M GuSCN and 1 mg/ml proteinase K, respectively. The two solutions are then mixed to a final GuSCN concentration of 3 M. The sandwich assay is then performed on this mixture. Still another alternative involves the identification and use of a lysis reagent useful for the release of nucleic acid from all organisms of interest (method 3, described above) The components of the buffered detergent solution, heating temperature, and time of heating may be adjusted for each organism individually or for the organisms in combination.

It will be obvious to one of ordinary skill in the art that, although the above discussion was set forth primarily in terms of nucleic acid hybridization assays, many other uses for these dipsticks are contemplated. Any member of a ligand pair can be attached to beads in the dipstick, and the dipstick can then be used to identify the corresponding ligand member. For example, antigens or antibodies could be attached to beads, as described above, in a dipstick, and then corresponding antibodies or antigens, respectively, could be identified. In a similar manner, other ligand systems, such as biotin and streptavidin, can be used.

The diagnostic methods of the present invention correlate well with traditional clinical diagnoses of BV. Illustrative patient samples used for comparative diagnostic testing were obtained from single, white females in their 20's at the Student Health Clinic at the University of Washington. The "normal" (i.e., BV-negative) patients were attending the clinic for routine examinations and showed no symptoms of vaginitis. pH was routinely measured at the time the samples were taken.

In these studies, a vaginal sample was obtained and analyzed in accordance with conventional techniques to obtain a conventional BV diagnosis. Subsequently, a vaginal wash was obtained, and a portion of this sample was mixed with concentrated 5 M GuSCN solution to yield a final concentration of 3 M GuSCN. The sample was concentrated onto nitrocellulose paper by slot blot techniques and then hybridized with a radiolabeled, G. vaginalis-specific oligonucleotide probe (i.e., the probe hybridizes specifically with 16S rRNA of G. vaginalis when challenged with more than 70 other potentially cross-reacting species that may be found in the normal microflora of the vagina). Hybridization of ³²P-labeled probe to each sample was determined by autoradiography, and levels of probe hybridization to samples and standards were compared.

A comparison of patient sample slot blot results with standards of known target nucleic acid concentrations allowed quantitative determination of the number of G. vaginalis cells in the patient samples. When the pH (greater than 4.5) and number of G. vaginalis cells (greater than or equal to approximately 2 x 10⁷) diagnostic criteria of the present invention were used in evaluating the slot blots, BV was detected with a sensitivity of 95.3% and a specificity of 98.6%. These diagnostic comparative results are set forth in Example 5 below.

The preferred diagnostic methods and kits of the present invention function through hybridization reactions, which constitute the established method for identifying specific nucleic acid sequences. Hybridization is based upon the pairing of complementary nucleic acid strands. When complementary single stranded nucleic acids are incubated in appropriate buffer solutions and conditions, complementary nucleotide sequences pair to form stable, double stranded molecules (i.e., the sequences hybridize).

The particular hybridization technique employed is not essential to the methods and kits of the present invention, and one of ordinary skill in the art will appreciate the variety of such techniques. Hybridization techniques are generally described in Hames et al. (eds.), "Nucleic Acid Hybridization, A Practical Approach", IRL Press, New York, 1985. As improvements are made in hybridization techniques, they will be readily applicable to the present invention. In addition, diagnostic dipstick and immunoassay technologies are known and employed in the art for a variety of purposes.

Once the target nucleic acid has undergone hybridization with the capture oligonucleotide probe, a second hybridization with a signal oligonucleotide probe must occur. In this manner, the presence of captured target nucleic acid is confirmed, and the amount of captured target nucleic acid may be quantified. The detection of successful or unsuccessful hybridizations may be accomplished in accordance with methods known to practitioners in the art.

Indirect quenching fluoroimmunoassay, double receptor fluoroimmunoassay, or protection fluoroimmunoassay are known assay formats that use antibodies directed against the fluorescer. These assays are based on competition for the fluorescer-labeled antigen by the antigen-specific antibody and the fluorescer-specific antibody.

As described in copending U.S. Patent Applications Serial Nos. 142,106, 444,872, 522,442, and 571,563, oligonucleotides covalently immobilized on polymer-coated beads or similar structures can serve as nucleic acid probes. Hybridization assays that detect the presence of specific target nucleic acid in complex biological samples can be conducted utilizing such immobilized oligonucleotide probes.

For the purposes of this description, the term "oligonucleotide" refers to a short nucleic acid sequence that is approximately 6 to 150 bases in length. Such oligonucleotides can be used as capture or signal probes in hybridization assays, and are preferably chemically synthesized using commercially available methods and equipment. For example, the solid phase phosphoramidite method can be used to produce short probes of between 6 and 100 bases having a molecular weight of less than 16,000 daltons. For the synthesis of oligonucleotides, see generally Caruthers et al., Cold Spring Harbour Symp. Quant. Biol. 47:411-18, 1982; and Adams et al., J. Am. Chem. Soc. 105:661, 1983. Activated oligonucleotides refer in general to oligonucleotides that have been reacted with a chemical compound and rendered chemically active. For the purposes of this description, the term "activatable" refers to the potential of a moiety to become chemically reactive.

When synthesizing an oligonucleotide probe for detection of a specific target nucleic acid, such as G. vaginalis RNA, DNA or the like, the choice of nucleotide sequence will determine the specificity of tests using such probes. For example, by comparing nucleic acid sequences from G. vaginalis isolates, one can select an oligonucleotide sequence for G. vaginalis detection that is either type-specific, species-specific or genus-specific. Comparisons of nucleic acid regions and sequences can be conducted using commercially available computer programs.

Oligonucleotide probes useful in the present invention are capable of hybridizing with a nucleic acid sequence specific for G. vaginalis; 16S G. vaginalis rRNA is a preferred G. vaginalis nucleic acid target for this purpose, because it is present in several thousand copies per cell. However, oligonucleotides complementary to sequences in the G. vaginalis genome or in G. vaginalis plasmids may also be employed. In addition, oligonucleotide probes capable of hybridizing with nucleic acid sequences specific for Candida spp. and Trichomonas vaginalis are described. Exemplary oligonucleotide probes useful in the present methods and kits are provided in the "Materials" section of this description and in Example 6.

The preferred capture oligonucleotides for use in the present invention are synthetic oligonucleotides from about 15 to about 100 bases in length. A spacer (linker) arm (i.e., a chemical moiety that extends or links other chemical groups, and preferably is a carbon chain containing from about 2 to about 12 carbon atoms, more preferably about 6 carbon atoms) containing a blocked amine group can be coupled, during oligonucleotide synthesis using conventional chemistry, to the 5'-hydroxyl group of an oligonucleotide. A primary amine is the preferred group for reaction with monofunctional or multifunctional reagents, and its attachment via a hexyl arm is preferred. Reagents useful for the attachment of spacer arms terminating in a primary amine are commercially available. Starting materials suitable for use in accordance with the present invention are known in the art.

Preferably, an oligonucleotide possessing a 5'-terminal structure, such as 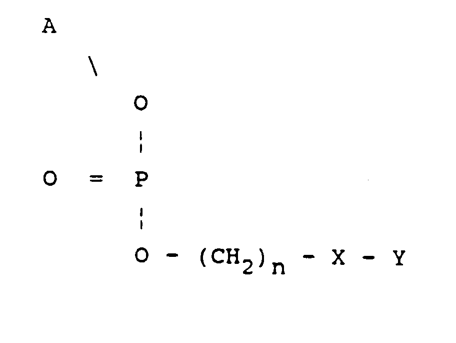 is employed, wherein n is 1-12, inclusive (n = 6 preferred); X is -NH- or -NHC:O(CH₂)ₘNH-, wherein m is 2-12, inclusive; Y is 4,6-dichlorotriazine (preferred) or a thiol (sulfhydryl)-reactive moiety; A is an oligonucleotide, ranging from about 9 to about 100 bases, preferably from about 15 to about 30 bases, with only the 5'-hydroxyl oligonucleotide moiety requiring modification for attachment. Alternatively, the 3' end of the oligonucleotide may be similarly modified to contain a reactive group.

Selected oligonucleotides are then activated with a monofunctional or multifunctional reagent. Exemplary reagents include homotrifunctional, heterotrifunctional, homobifunctional, and heterobifunctional reagents. Selected oligonucleotides may be activated and linked to polymer-coated solid supports according to the following chemistries. An amine-tailed oligonucleotide can be activated with a monofunctional or multifunctional reagent (e.g., cyanuric chloride). The alkylamino dichlorotriazine formed in the activation step is reactive toward the amine-containing polymer bound to the solid support.

Although cyanuric chloride, a homotrifunctional reagent, is preferred, other activating reagents can be used. For example, N-succinimidyl-4-(iodoacetamido)-benzoate (SIAB) is a suitable heterobifunctional reagent, and disuccinimidyl suberate is a suitable homobifunctional reagent. If solid support carboxyl groups are involved, the heterobifunctional reagent 1-ethyl-3-(dimethylaminopropyl)-carbodiimide can be used. Other similar monofunctional and multifunctional (heteromultifunctional and homomultifunctional) reagents are suitable for use in the practice of the present invention.

The oligonucleotide activation and linking chemistries result in the selective activation of an amino group on an oligonucleotide, without modification of any of the purine and pyrimidine bases of the oligonucleotide. Specifically, the preferred chemistry employs cyanuric chloride (2,4,6,-trichloro-1,3,5, -triazine). Oligonucleotides possessing a 5' or 3' tethered (via a hexyl arm) nucleophilic amine moiety (or internal aminoalkyl groups substituted on pyrimidine or purine bases) are reacted with an excess, preferably 50- to 200-fold, of recrystallized cyanuric chloride at, preferably, 19-25°C in a part organic solvent, such as N-methyl pyrrolidone, for 1 to 2 hours.

The unreacted cyanuric chloride can be removed by exclusion chromatography or ultrafiltration. The treated solid support and activated oligonucleotide are then conjugated. Specifically, they are mixed together and preferably incubated at from about 20 to 50°C for about 1 to 24 hours. The residual (unreacted) amines on the bead surface can be capped (blocked) with an agent, such as succinic anhydride, preferably in N-methyl pyrrolidone in the presence of an appropriate base, such as sodium borate, to render the surface compatible (negatively charged) for nucleic acid hybridization. It should be noted that the solid support may be chemically modified to produce a positive, negative, or neutral surface charge.

The target nucleic acid is usually a polynucleotide with an average length ranging from about 20 to about 20,000 bases or nucleotides in length. Suitable conditions for hybridization relate to stringency conditions wherein base-pairing mismatching is minimized or non-existent. The degree of acceptable mismatching is dependent upon the specificity required, in a manner recognized by a practitioner in the art.

Signal oligonucleotide probes useful in accordance with the preferred methods and kits of the present invention are oligonucleotides conjugated to or conjugable with detectable labels. Various labels can be used in hybridization assays of this invention. Such labels act as reporter groups for detecting duplex formation between the target nucleic acid and its complementary signal sequence. A reporter group as used herein is a group having a physical or chemical characteristic that can be measured or detected. Detectability may be provided by such characteristics as enzymatic activity, color change, luminescence, fluorescence, or radioactivity, or it may be provided by the ability of the reporter group to serve as a ligand recognition site. Any haptenic or antigenic compound can be used in combination with a suitably labeled antibody for this purpose.

Exemplary enzymes of interest as reporter groups are hydrolases, particularly phosphatases, esterases, ureases, and glycosidases, oxidoreductases, particularly peroxidases, and the like. Fluorescent compounds include fluorescein and its derivatives, rhodamine and its derivatives, dansyl, umbelliferone, and the like. Chemiluminescers include luciferin, luminol, oxetane-diones, and the like. The above list is illustrative only, and the choice of label depends on sensitivity requirements, ease of conjugation with the probe, stability requirements, and available instrumentation.

The extent of hybridization may be quantified using a method or technique of fluorescent quenching, described in copending U.S. Patent Application Serial No. 558,967. Dipstick (i.e., insoluble) diagnostic format fluorescent quenching employs a solid support, such as nylon, that fluoresces when irradiated with ultraviolet light (240 to 400 nanometers (nm)). Colorimetric insoluble enzymatic product deposited on the solid support during the preferred assay procedure quenches the fluorescence of the solid support, thereby providing a means of quantifying the amount of product using commercially available fluorometers. The solid supports described herein do not require a plurality of fluorescent chromophoric groups to be bound thereto, but rather rely on the intrinsic or natural fluorescence of the polymeric material forming the solid support. The irradiation and detection of fluorescence in a hybridization assay methodology utilizing this type of support are also not dependent on the use of narrow or specific wavelengths of ultraviolet or visible light.

For example, a sandwich may be formed in which a target nucleic acid is hybridized to the solid support; a signal biotinylated-oligonucleotide is then hybridized to the target nucleic acid; and reporter enzyme conjugated to streptavidin is bound to the biotinylated oligonucleotide. After sandwich formation, reporter enzyme product is allowed to deposit or accumulate on the surface of the solid support. In most cases, the quantity of enzymatic product produced is directly proportional to the quantity of captured target nucleic acid. The solid support is then irradiated with an ultraviolet light source (240 to 400 nm) and the resultant fluorescence is determined with a fluorometer. The intensity of the measured fluorescence is inversely proportional to the quantity of enzymatic product deposited on the solid support. Alternatively, if the reporter enzyme product is colored, product deposited or accumulated on the surface of the bead can be qualitatively and/or quantitatively determined. Quantitative determinations may be performed visually or by an instrument capable of analyzing and/or measuring gradations of color, either directly or using shades of grey. Such quantitative determinations generally include a comparison to standards.

If the fluorescence quenching method is used, the only required property of an enzymatic product useful in quantifying the amount of captured target nucleic acid is the ability to quench or mask the fluorescence of the solid support. Any type of enzyme which produces a colorimetric product can be utilized in the fluorescent quenching assay. Exemplary enzymes include horseradish peroxidase (HRP), alkaline phosphatase (AP), beta-galactosidase and the like. Representative substrates for each exemplary enzyme include 4-methoxynaphthol (4MN), 5-bromo-4-chloroindoyl-3-phosphate/nitroBlue tetrazolium (NBT), and o-nitrophenyl-beta-D-galactopyranoside (ONG), respectively.

Since the quantity of enzymatic product produced is proportional to the quantity of captured target nucleic acid, and the quenching of fluorescence by the colored product is proportional to the quantity of product produced, a quantitative determination of captured target nucleic acid can be made. In most cases, the relation between captured target nucleic acid and fluorescence quenching is linear.

In this embodiment, the solid support must necessarily possess some type of fluorescence when irradiated by ultraviolet or visible light. The fluorescence can be intrinsic or inherent to the material composing the solid support, or fluorescent compounds can be bound (either covalently or non-covalently) to the solid support during the manufacturing or derivitization process. Exemplary fluorescent compounds include fluorescein, Texas Red, rhodamine and the like.

Dipstick format diagnostics of the present invention may be evaluated by visual assessment only. In this embodiment, the bead will turn from its natural color to an indicator color, such as blue, to indicate positive results. For example, a dipstick might be designed to turn an indicator color visible to the eye when a sufficient amount of a target nucleic acid is present. This embodiment of the present invention will not require irradiation of the sample with ultraviolet light or detection with a fluorometer.

Alternatively, direct determination or quantitation of fluorescent enzymatic product may be employed in a soluble detection format (e.g., 96 well format) of the present invention. To obtain accurate results, however, the fluorescent enzymatic product must be measured in an environment that neither quenches the product nor is fluorescent itself. Since the preferred solid supports described herein possess a very high intrinsic fluorescence in their native state, it is not possible to accurately measure a fluorescent soluble enzymatic product in the presence of the preferred solid support. Such a solid support must therefore be removed from the substrate solution or the solution decanted and placed in a separate vessel to allow accurate determination of fluorescence.

Alternatively, for fluorescent signal detection, colored or coated polymeric beads may be employed. Coloring the preferred solid support with a dye of any color significantly reduces or quenches the intrinsic fluorescence thereof. Coating serves to mask any intrinsic fluorescence of a particular solid support. This reduction in fluorescence permits the solid support to be present during soluble enzymatic product fluorescence measurement, thereby obviating the need to transfer solutions or solid supports.

If a bead or solid support (preferably dyed or colored) remains in solution during fluorescent signal detection, bead-to-bead or solid support-to-solid support fluorescence must be relatively uniform. The standard deviation of bead-to-bead intrinsic fluorescence should not exceed the standard deviation of the assay (typically 1 to 10%) or of the detected fluorescence (typically 1 to 5%), in order to fully utilize the potential of the fluorescent substrate. This procedure is used in the microtiter well format of the present invention, where a soluble fluorescent product is measured directly in the presence of the colored bead. Less meaningful measurements are obtained when deviations greater than those set forth above occur.

Virtually any color of dichlorotriazine, azo, or other permanent dyes will reduce the intrinsic fluorescence of the nylon approximately 500-fold. Exemplary dye colors are black, blue, red, green, yellow, purple, orange and the like, with the only requirement with respect to dye color being that the dye not fluoresce at the same wavelength as the fluorescent enzymatic product.

In addition to quenching the natural fluorescence of the preferred solid support, coloring the supports allows the development of solid supports with different capture oligonucleotides or different target nucleic acid specificities that are distinguishable by color. The ability to distinguish different solid supports by color has the following advantages: 1) quality control can be enhanced, because solid supports possessing different specificities can be identified and distinguished; and 2) contrast between a colorimetric enzymatic product and the surface of the solid support can be maximized. This allows a greater level of sensitivity to be achieved when assay results are determined by visual inspection.

The kits of the present invention can be used as part of a semi-automated method for BV diagnosis in accordance with the present invention. Such kits would provide for the detection of G. vaginalis when present at or above the level characteristic of BV, and may include a sample obtaining means, such as a vaginal swab; a pH indicating means, such as pH paper; lysis buffer/hybridization solution and ligand incubation solution; and G. vaginalis cell number indicating means, such as a diagnostic dipstick. This kit may be used in conjunction with an incubation apparatus (such as a heating block), an automated assay device and/or a bead reader.

A practitioner using the semi-automated method of the present invention for the diagnosis of BV will follow a procedure substantially as described below:
1) Obtain a vaginal swab and apply the swab to provided pH paper.
2) Insert the swab into a tube containing lysis solution and soak for approximately five minutes or less.
3) Squeeze out the swab, and optionally place the tube into a well of a heating block, heating at approximately 65°C for approximately five to twenty minutes.
4) Add 5 M GuSCN solution to a final concentration of 3 M.
5) Transfer the solution to the first sample well in an automated dipstick processor, which automatically completes the assay development in approximately 30 minutes.
6) Visually determine whether a colored substance has been deposited on the bead.

In this semi-automated methodology, step 1 corresponds to determining pH of a vaginal sample obtained from the patient. Step 2 may use a proteinase K lysis buffer. Steps 2-6 constitute determining whether the critical G. vaginalis cell number is equalled or exceeded by the patient sample.

These methods and kits are advantageous in that they provide accurate BV diagnosis rapidly and reproducibly, without a requirement for highly skilled, labor-intensive analysis. The methods of the present invention may be accomplished in 6 hours or less, and preferably are accomplished in 1 hour or less.

Also, the methods and kits of the present invention eliminate the need for skill in identifying clue cells or evaluating wet mounts. Consequently, the methods of the present invention could be practiced and the kits of the present invention could be used by individuals without the aforementioned skills. Specifically, a laboratory technician or physician's assistant could employ the methods and kits of the present invention with virtually no special training. Another ramification of using the methods and kits of the present invention is that the subjectivity involved in wet mount and Gram stain analysis is replaced with more objective procedures. Specifically, analyst-to-analyst variation is eliminated through the use of the present invention.

In the physician's office setting, physical examinations and wet mounts are primarily relied upon to diagnose BV. These methodologies are less accurate than the gold standard method, but the gold standard method is generally considered too complicated to perform in an office environment. The methods and kits of the present invention can be utilized in the office setting to achieve diagnostic results comparable to those provided by the gold standard method or the physical examination/wet mount method.

In an alternative embodiment, patient samples may be collected, processed and analyzed to simultaneously detect the presence of Gardnerella vaginalis, Candida spp., and Trichomonas vaginalis. Samples are collected from symptomatic females presenting with vaginal complaint who have not been treated with anti-bacterial or anti-fungal medication within the week prior to sample collection and who have not douched within 24 hours.

In a preferred procedure, a sterile swab is used to obtain vaginal fluid samples. Dacron swabs with pre-scored handles are particularly preferred for sample collection. Samples are obtained by twisting or rolling the swab against the vaginal wall two or three times, ensuring that the entire circumference of the swab has touched the vaginal wall. The swab is then placed in a sample collection tube, the pre-scored handle of the swab is broken, and the tube is capped. The unlubricated speculum is removed from the patient, and the vaginal pH is determined by touching a pH indicator strip to the speculum.

In a protocol for immediate sample preparation, the swab/sample is transported immediately at room temperature for processing and analysis. If the swab/ sample cannot be immediately processed, the swab/sample is held at 0°C to 8°C for four hours or at room temperature for one hour.

For processing, lysis solution is added to the swab/sample, which is then swirled or agitated in the lysis solution for about 10-15 seconds. The tube containing the swab and lysis solution is heated at 85°C for 5 minutes, and hybridization solution is mixed with the sample. At this point, samples may be stored for up to 24 hours at room temperature.

The swab contents are expressed by twirling the swab against the side of the tube, and the solution remaining in the tube may then be processed on an automated instrument. Generally, the solution remaining in the tube is filtered prior to further processing. In a preferred embodiment, the sample is placed in the first well of a reagent cassette or multi-cavity container, and the semi-automated instrument moves a dipstick through each well of the cassette, thereby processing the sample. A preferred dipstick contains five beads -- a procedural control, a negative control, a bead with Gardnerella vaginalis-specific capture probe, a bead with Candida spp.-specific capture probe, and a bead with Trichomonas vaginalis-specific capture probe. It is preferred that the detection of these three organisms be achieved through a colorimetric signal system, wherein the presence of color on a test bead at the end of automated sample processing is indicative of a detectable level of nucleic acid from that target organism. The intensity of color on a bead can be estimated either visually or by a measuring device, such as an image analyzer or reflectometer. The procedural control ensures that the procedure has been correctly performed and serves as a reagent quality check. The negative control evaluates non-specific binding to the beads.

In the experimental section below, Example 1 describes quantitation of horseradish peroxidase insoluble product on 3/32nd inch nylon beads using the fluorescence quenching technique. Example 2 describes the quantitation of alkaline phosphatase insoluble product on 3/32nd inch nylon beads using the fluorescence quenching technique. Example 3 describes a comparison of direct fluorescence using soluble 4-methyl-umbelliferone in the presence of black and natural colored nylon beads. Example 4 describes the reduction in fluorescence of nylons beads by dying the beads with a multiplicity of different colors. Example 5 illustrates the correlation between the diagnostic criteria employed in the practice of the present invention and conventional BV diagnostic techniques. Example 6 describes methods and kits for simultaneous detection of Gardnerella vaginalis, Candida spp., and Trichomonas vaginalis target nucleic acid in a complex biological sample.

The following Materials and Procedures sections pertain to the above-summarized Examples 1-5.

### MATERIALS

### Solutions:

APB buffer is 0.18 M NaCl, 0.05 M Tris-HCl, pH 7.6, 5 mM EDTA, and 0.5% (v/v) Tween® 20.

TMNZ buffer is 0.05 M Tris, pH 9.5, 1 mM MgCl₂, and 0.5 mM ZnCl₂.

FW (filter wash) is 0.09 M sodium chloride, 50 mM Tris, pH 7.6, and 25 mM EDTA.

SDS/FW is FW and 0.1% (w/v) sodium dodecyl sulfate (SDS).

HRP (horseradish peroxidase) substrate solution is 0.1 M sodium citrate, pH 6.5, 0.2 M sodium phosphate, 0.5 mg/ml 4-methoxy-1-naphthol, 0.02 mg/ml 3-methyl-2-benzothiazolinone hydrazone and 0.0135% (v/v) hydrogen peroxide.

AP (alkaline phosphatase) substrate solution is 1 mM 5-bromo-4-chloroindoyl-3-phosphate, 1 mM nitroBlue tetrazolium, and 0.01% (v/v) Tween® 20 in TMNZ.

5 M GuSCN is 5 M guanidinium thiocyanate, 83.5 mM Tris, pH 8.0, 8.35% formamide, and 16.7 mM EDTA.

3 M GuSCN is 3 M guanidinium thiocyanate, 50 mM Tris, pH 8.0, 5% formamide, and 10 mM EDTA.

Proteinase K lysis solution is 1 mg/ml proteinase K, 0.5% (w/v) SDS, and 5% N-lauroylsarcosine (sarcosyl).

Lysis and hybridization solution is 3 M guanidinium thiocyanate (GuSCN), 50 mM Tris, pH 7.6, 2% sarcosyl, and 25 mM EDTA.

Hybridization/slot blot solution is 90 mM Tris, pH 8.0, 0.6 M NaCl. 10 mM EDTA, 0.5% SDS, 5X Denhardt's (1X Denhardt's = 0.02% (w/v) Ficoll, 0.02% (w/v) polyvinylpyrrolidone, and 0.02% (w/v) bovine serum albumin, Fraction V), 30% formamide, and 100 µg/ml homomix (hydrolyzed RNA yielding RNA fragments of ≈ 5-15 bases in length).

CAP buffer is 0.1 M sodium citrate, pH 6.5 and 0.2 M sodium phosphate.

The fluorescent substrate for alkaline phosphatase is 0.02 mM 4-methyl-umbelliferyl phosphate, 0.05 M Tris, pH 9.5, 1 mM MgCl₂, and 0.5 mM ZnCl₂.

### Oligonucleotide sequences:

Gv003:  5'AGA-CGG-CTC-CAT-CCC-AAA-AGG-GTT3'
Ca1004:   5'TTC-CTC-GTT-AAG-GTA-TTT-ACA-TTG-TAC3'
Up33:   5'GAA-TTA-CCG-CGG-CTG-CTG-G3'
Gv009:  5'GGC-CCC-ACA-TCC-AGC-GTC-CAC-CGT3'
UP041:  5'CTG-CTG-CCT-CCC-GTA-GGA-GT3'

UP007: 5'GTA-TTA-CCG-CGG-CTG-CTG3'
Trv12: 5'ATC-CTN-AAA-GAC-CCG-AAG-CCT-GTC3'
UP28: 5'CGA-CGG-GCG-GTG-TGT-ACA-A3'

In sequence Trv12, "N" is A, C, G or T/U, or an unknown or other nucleotide.

Poly(ethyleneimine) may be purchased from Polysciences (Warrington, PA).

Burnished or unpolished nylon beads may be purchased from Precision Ball Company (Chicago, IL) and The Hoover Group (Sault St. Marie, MI).

Triethyloxonium tetrafluoroborate, hexanediamine, phenylenediamine, succinic anhydride and N-methyl-pyrrolidone, Cibacron Brilliant Red, Cibacron Brilliant Yellow, Mordant Orange, Fast Blue BB, Reactive Blue 2, Mordant Brown 4, and Reactive Black may be purchased from Aldrich Chemical (Milwaukee, WI).

N-succinimidyl 4-(iodoacetamido)-benzoate (SIAB) and Tween® 20 may be purchased from Pierce (Rockford, IL).

Guanidinium thiocyanate (GuSCN) may be purchased from Kodak (Rochester, NY).

### PROCEDURES

### Oligonucleotide synthesis:

Oligonucleotides complementary to conserved regions or hypervariable regions of the 16S ribosomal RNA of G. vaginalis, Trichomonas vaginalis, or Candida albicans are synthesized using phosphoramidite chemistry utilizing an ABI 380B, a Milligen 7500 automated DNA synthesizer, or a similar instrument. The oligonucleotides are prepared using the standard phosphoramidite chemistry supplied by the vendor or H-phosphonate chemistry. Appropriately blocked dA, dG, dC, and dT phosphoramidites are commercially available in these forms, and synthetic nucleosides may readily be converted to the appropriate form. Oligonucleotides are purified by adaptations of standard methods. Oligonucleotides with 5'-trityl groups are chromatographed on HPLC using a 12 µm, 300 Å Rainin (Woburn, MA) Dynamax C-8 4.2 x 250 mm reverse phase column using a gradient of 15% to 55% MeCN in 0.1 N Et₃NH⁺OAc⁻, pH 7.0, over 20 minutes. When detritylation is performed, the oligonucleotides are further purified by gel exclusion chromatography. Analytical checks for the quality of the oligonucleotides are conducted with a Toso-Haas DEAE-NPR column at alkaline pH and by polyacrylamide gel electrophoresis (PAGE).

### Preparation of a polymer-coated nylon bead:

25,000 3/32 inch diameter unpolished nylon beads are placed in a flask containing 1800 ml of 100% anhydrous N-methyl-pyrrolidone and mixed for 5 minutes at ambient temperature. 200 ml of 1 molar triethyloxonium tetrafluoroborate in dichloromethane are added and the mixture is stirred for 30 minutes at ambient temperature. The beads are then decanted and washed quickly with four 500 ml changes of 100% N-methyl-pyrrolidone. The beads are then transferred to a solution consisting of 3% (w/v) 10,000 MW poly(ethyleneimine), prepared from a 30% aqueous solution of poly(ethyleneimine), in N-methyl-pyrrolidone and stirred for 12 to 24 hours at ambient temperature. The beads are washed with 2000 ml N-methyl-pyrrolidone, 1000 ml SDS/FW and finally 10 x 2 liter distilled water. The beads are then dried under high vacuum for 4 to 5 hours without the use of heat. The amine content of the beads may be determined by reaction with picrylsulfonic acid.

### Preparation of cyanuric chloride-derived oligonucleotides:

10 to 1000 µg of 5'-amine-linked oligonucleotide spiked with a small amount of the same oligonucleotide labeled at its 3' end with ³²P are reacted with an excess of recrystallized cyanuric chloride in 10% (w/v) N-methyl-pyrrolidone in an alkaline buffer (pH 8.3 to 8.5, preferably) at ambient temperature for 30 to 120 minutes. The final reaction conditions are 0.15 M sodium borate at pH 8.3, 2 mg/ml recrystallized cyanuric chloride and 500 µg/ml appropriate aminohexyl oligonucleotide. The unreacted cyanuric chloride is removed by size exclusion chromatography on a G-50 Sephadex column (Pharmacia, Uppsala, Sweden).

Cyanuric chloride-derived oligonucleotides and poly(ethyleneimine) coated-nylon beads (described above) are placed in a volume of 0.1 M sodium borate, pH 8.3 equal to the volume of the beads at 4°C. The purified cyanuric chloride-derived oligonucleotide is then added to the beads, and the mixture is vigorously agitated at ambient temperature for 60 minutes. The beads are then washed twice with 0.1 M sodium borate (pH 8.3). Succinic anhydride is then added at a concentration of 10 mg/ml in 90% (w/v) N-methyl-pyrrolidone and 10% (w/v) 1 M sodium borate (pH 8.3) having a volume three times that of the volume of the beads. The reaction is allowed to proceed for 1 hour at ambient temperature. The beads are then washed 3 times with 250 ml of 100% (v/v) N-methyl-pyrrolidone, twice with distilled water, 5 times with 250 ml SDS/FW and then 4 times with 1 liter of distilled water. Beads are stored dry or in 25 mM EDTA. Radio-activity per bead may be determined by liquid scintillation counting, allowing the amount of capture oligonucleotide per bead to be calculated.

### Lysis of bacteria and hybridization conditions:

1 x 10⁸ G. vaginalis cells are incubated with 100 µl of Proteinase K lysis solution for 20 minutes at 65°C. This preparation is then combined with 150 µl 5 M GuSCN. Five to eight 5-fold serial dilutions are made of the starting lysate. Biotinylated probe is added to the lysates to a final concentration of 100 ng/ml. The solutions are then incubated with the derived nylon beads (covalently immobilized with about 0.1 µg of G. vaginalis oligonucleotide probe (capture probe)) for about 10 minutes to 1 hour at ambient temperature with mild agitation. The solid supports are then washed two times with SDS/FW. Streptavidin/HRP conjugate is added to a final concentration of 1 µg/ml (based on streptavidin) in SDS/FW and incubated about 5 to 15 minutes at ambient temperature with mild agitation. The beads are washed three times with SDS/FW and then once with CAP buffer. The beads are combined with 0.3 ml of 4-methoxy-naphthol substrate solution (as described above), and the reaction is allowed to proceed for 15 minutes at ambient temperature. At this stage of the procedure, the capture probe bead will become colored if G. vaginalis nucleic acid is detected. The beads are then quickly washed once with SDS/FW, once with distilled water and allowed to air dry in the dark.

### Quantitative determination of the extent of hybridization (capture of target nucleic acid) using insoluble substrates for either horseradish peroxidase or alkaline phosphatase:

After the completion of the sandwich assay on the solid support, herein 3/32 inch nylon beads, and the deposition of the insoluble, colored substrate product onto the surface of the bead by either HRP or alkaline phosphatase, the quantity of target nucleic acid captured is determined by fluorescence quenching. The beads are dried for 15 to 30 minutes at ambient temperature and then individually placed in a round-bottom, opaque white microtiter plate (Dynatek Laboratories, Chantilly, VA). The beads are then read using a fluorometer (Fluoroskan II, Flow Laboratories, McLean, VA) or equivalent instrument, in which excitation is at 350 nm and emission is at 456 nm. The beads possess an intrinsic fluorescence of about 800 RFUs, and the presence of the colorimetric substrate product effectively quenches the intrinsic fluorescence. Lower indicated fluorescences correlate with greater quantities of captured target nucleic acid.

### EXAMPLE 1

Example 1 describes the quantitation of signal in a typical sandwich assay format, in which a target nucleic acid is sequestered and then detected using a colorimetric insoluble enzymatic product obtained using a horseradish peroxidase system. The colored product (i.e., results) may be evaluated by measuring quenching of the natural fluorescence of the bead.

1 mg/ml proteinase K lysis solution is used to lyse 1 x 10⁸ G. vaginalis cells in 100 µl volumes for 20 minutes at 65°C. The lysate is adjusted to 3 M GuSCN by adding 1.5 volumes of 5 M GuSCN. A biotinylated 24-mer oligonucleotide probe complementary to conserved regions of bacterial 16S rRNA (signal probe) is added to a final concentration of 100 ng/ml.

5-fold serial dilutions of the lysates are made using 3 M GuSCN hybridization solution containing the biotinylated signal oligonucleotides. Each dilution (200 µl) is then incubated for 30 minutes at ambient temperature with 3 white nylon beads having 0.1 µg of G. vaginalis-specific oligonucleotide probe (capture probe) covalently immobilized thereon. The solid supports are washed twice with SDS/FW at ambient temperature and then incubated with 1-2 µg/ml of streptavidin/horseradish peroxidase (SA/HRP) conjugate in SDS/FW for 5 minutes at ambient temperature. The solid supports are washed with SDS/FW, and then the presence of peroxidase is determined by incubating the beads for about 10-30 minutes with the HRP substrate solution (4MN) to form an insoluble blue product. The beads are then washed once with SDS/FW, wicked dry, and placed in a white, round bottom 96-well microtiter plate (Dynatek Laboratories, Chantilly, VA). The beads are read using a fluorometer (Fluoroskan II, Flow Laboratories, McLean, VA) in which excitation is at 350 nm and emission is at 456 nm. Results of such testing will show a decrease in RFUs with an increase in bacterial cell number.

### EXAMPLE 2

Example 2 describes the quantitation of signal in a typical sandwich assay format, in which a target nucleic acid is sequestered and then detected using a colorimetric insoluble enzymatic product obtained using an alkaline phosphatase system.

Proteinase K lysis solution is used to lyse 1 x 10⁸ G. vaginalis cells in 250 µl volume at 65°C for 20 minutes. The lysate is adjusted to 3 M GuSCN by the addition of 1.5 volumes of 5 M GuSCN. A biotinylated 24-mer oligonucleotide probe complementary to conserved regions of bacterial 16S rRNA (signal probe) is added to a final concentration of 100 ng/ml.

5-fold serial dilutions of the lysates are made using 3 M GuSCN hybridization solution containing the biotinylated signal oligonucleotides. Each solution is then incubated for 30 minutes at ambient temperature with 3 nylon beads having 0.1 µg of G. vaginalis-specific oligonucleotide probe (capture probe) covalently immobilized thereon. The solid supports are then washed twice with SDS/FW at ambient temperature and incubated with 10 ng/ml of streptavidin/alkaline phosphatase (SA/AP) conjugate in APB buffer for 5 minutes at ambient temperature. The solid supports are washed with APB buffer. The presence of alkaline phosphatase may be determined by incubating the beads with the TMNZ substrate solution for 1-4 hours to form an insoluble formazan product. The beads are then washed once with SDS/FW, wicked dry, and placed in a white, round-bottom 96-well microtiter plate (Dynatek Laboratories, Chantilly, VA). The beads are then read using a fluorometer (Fluoroskan II, Flow Laboratories, McLean, VA) in which excitation is at 350 nm and emission is at 456 nm. Results of such testing will show a decrease in RFUs with an increase in bacterial cell number.

### EXAMPLE 3

Example 3 demonstrates the use of nylon solid supports in a sandwich assay format, in which a target nucleic acid is sequestered and then detected using an assay format based on detecting a fluorescent product in the presence of beads in a microtiter well embodiment.

Proteinase K lysis solution is used to lyse 1 x 10⁸ G. vaginalis cells in 100 µl volumes at 65°C for 20 minutes. The lysate is adjusted to 3 M GuSCN by the addition of 1.5 volumes of 5 M GuSCN. A biotinylated 24-mer oligonucleotide probe complementary to conserved regions of bacterial 16S rRNA (signal probe) is added to a final concentration of 100 ng/ml.

5-fold serial dilutions of the lysates are made using 3 M GuSCN hybridization solution containing the biotinylated signal oligonucleotides. Each solution is then incubated for 30 minutes at ambient temperature with 2 black nylon beads prepared by The Hoover Group (Sault St. Marie, MI) and 2 natural colored nylon beads, each having 0.1 µg of G. vaginalis-specific oligonucleotide probe (capture probe) covalently immobilized thereon. The solid supports are washed with SDS/FW at ambient temperature, followed by washing with 0.1% (v/v) Tween® 20, 1 mM MgCl₂, 0.01 M Tris-HCl, pH 8.0 (APB) and incubation with 0.4 µg/ml of streptavidin/alkaline phosphatase (SA/AP) conjugate in APB for 5 minutes at ambient temperature. The solid supports are then washed 5 times with APB, once with TMNZ, and then the presence of alkaline phosphatase may be determined by incubating the nylon beads individually with 150 µl of 0.5 mM 4-methyl-umbelliferyl phosphate (4-hydroxymethyl coumarin) in black microtiter well strips (Dynatek, Laboratories, Chantilly, VA). Incubation is for 30 minutes at 37°C. The plates are then directly read using a Fluoroskan II fluorometer (Flow Laboratories, McLean, VA) using an excitation wavelength of 360 nm and an emission wavelength of 456 nm.

As a result of the very high intrinsic fluorescence associated with the virgin (natural colored) nylon beads (800-900 RFUs), a higher level of cells (target nucleic acid) will be detected using the black nylon beads, as compared to the natural colored nylon bead solid supports, when the quantitation is conducted in the presence of the beads. The black nylon beads, which possess a decreased intrinsic fluorescence, will allow the sensitive detection of G. vaginalis 16S rRNA using a fluorescence-based signal system. A control in which the natural colored bead is physically removed from the solution is read, and is expected to indicate the same level of detection of G. vaginalis as when the solution is read in the presence of the black bead.

### EXAMPLE 4

Example 4 describes the coloring or dying of 3/32nd inch nylon beads with a multiplicity of dyes, and the resultant reduction of the intrinsic fluorescence of the nylon bead.

Approximately 500 mg of Mordant Red, Reactive Blue 2, Mordant Brown 4, Cibacron Brilliant Red, Cibacron Brilliant Yellow, Reactive Black, or Mordant Orange were dissolved in 50 ml of 50% (v/v) N-methyl-pyrrolidone and 0.2 M sodium borate (pH 8.3) and incubated with 1000 3/32nd inch nylon beads for 24 hours at ambient temperature and 1 hour at 65°C. The beads were then washed with 10 changes of 50 ml of 100% N-methyl-pyrrolidone and 5 changes of 50 ml distilled water. The beads were then dried under high vacuum for 25 hours. Eight beads from each color group were then placed in a round-bottomed, white microtiter plate.

The plates were then directly read using a Fluoroskan II fluorometer (Flow Laboratories, McLean, VA) using an excitation wavelength of 360 nm and an emission wavelength of 456 nm. The results are shown in Table 2 below.

**TABLE 2**

| DYED BEADS AND FLUORESCENT SIGNAL | |
|---|---|
| Color | RFUs |
| Native (white) | 2000 |
| Mordant Brown | 3.5 |
| Cibacron Red | 110 |
| Cibacron Yellow | 130 |
| Reactive Black | 2.5 |
| Fast Blue BB | 10 |
| Mordant Orange | 30 |
| Reactive Blue | 3.0 |

Coloring the nylon bead significantly reduced the intrinsic fluorescence of the nylon bead, thereby rendering the beads compatible with fluorescence-based assays when dyed with Mordant Brown, Reactive Black, Fast Blue BB, and Reactive Blue.

### EXAMPLE 5

Example 5 illustrates the correlation between the diagnostic criteria employed in the practice of the present invention and conventional BV diagnostic techniques.

Vaginal washes were collected from 43 women determined to be positive for BV by standard clinical criteria and from 70 BV-negative women. These samples were obtained from women visiting the Student Health Clinic at the University of Washington. The women were 92% Caucasian and 8% Asian, with a median age of 23.8 years. Eighty percent of the women tested were unmarried. None were pregnant. Women were determined to be positive for BV by employing the current gold standard diagnostic criteria for the disease. BV-negative women were negative for BV according to the gold standard criteria, and had no obvious signs or symptoms of vaginitis.

Vaginal pH was measured at the time of examination. 3 ml of sterile Hank's balanced salt solution (Flow Laboratories, McLean, VA) were injected into a patient's vagina and withdrawn to a sterile test tube. 0.25 ml of this vaginal wash were added to 0.5 ml of 5 M GuSCN.

Aliquots of 50 and 10 µl of each lysate were diluted to a final volume of 200 µl in 3 M GuSCN and loaded onto Nytran filters (Schleicher & Schuell, Keene, NH) using a Schleicher & Schuell Minifold II slot blot template. Serial dilutions of purified 16S rRNA from G. vaginalis were included on each Nytran filter to provide a standard for quantitation. The filters were baked at 80°C for 2 hours to lyse the G. vaginalis cells and to fix released RNA onto the Nytran filters. The filters were then hybridized with 1 x 10⁶ cpm/ml of ³²P-labeled GV003, an oligonucleotide specific for G. vaginalis 16S rRNA (i.e., it hybridizes specifically with the 16S rRNA of G. vaginalis when challenged with over 70 other potentially cross-reacting species of normal vaginal microflora). Hybridization was performed in hybridization/slot blot solution in Seal-a-Meal bags (Dazey). Filters were washed three times with SDS/FW at 55°C and then were exposed to Kodak X-OMAT AR film, which was developed by standard procedures.

The amount of G. vaginalis cells present in the sample was quantitated by comparing the signal intensities obtained from the standards on the filters and those obtained from patient samples. These comparative results could be expressed in terms of cell number, as a result of an additional slot blot experiment. A serial dilution of the same 16S rRNA standard was loaded on a Nytran filter using a Minifold II slot blot template. Serially diluted lysates from three different G. vaginalis cultures having known cell numbers (measured by microbiological methods described in the references set forth above) were also loaded on the filter. The slot blots were baked at 80°C for 2 hours and hybridized with a ³²P-labeled, G. vaginalis-specific probe, GV003. Filters were washed and exposed to Kodak X-OMAT AR film, which was developed by standard procedures. Visual examination of the resultant autoradiogram allowed the assignment of a correlation between a given amount of 16S rRNA and a known number of G. vaginalis cells.

This correlation was used in combination with autoradiograms of the patient sample slot blots to obtain the cell numbers set forth in Table 3 below.

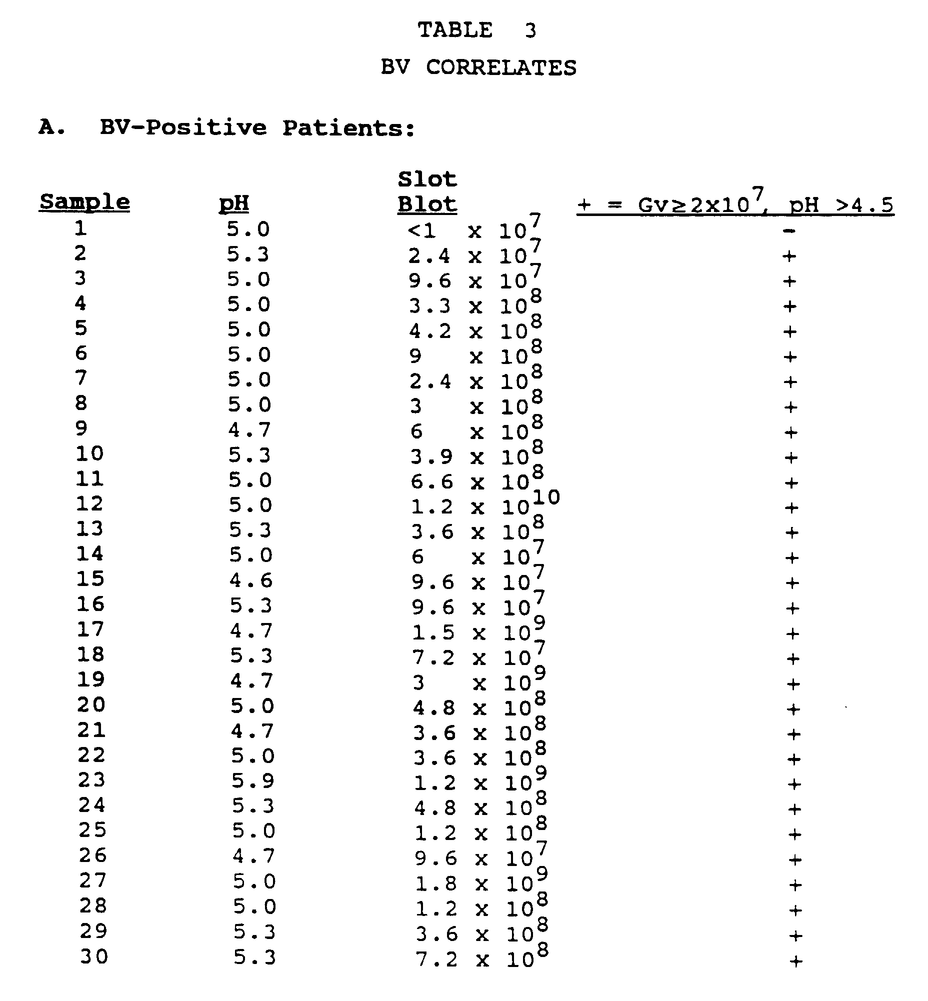

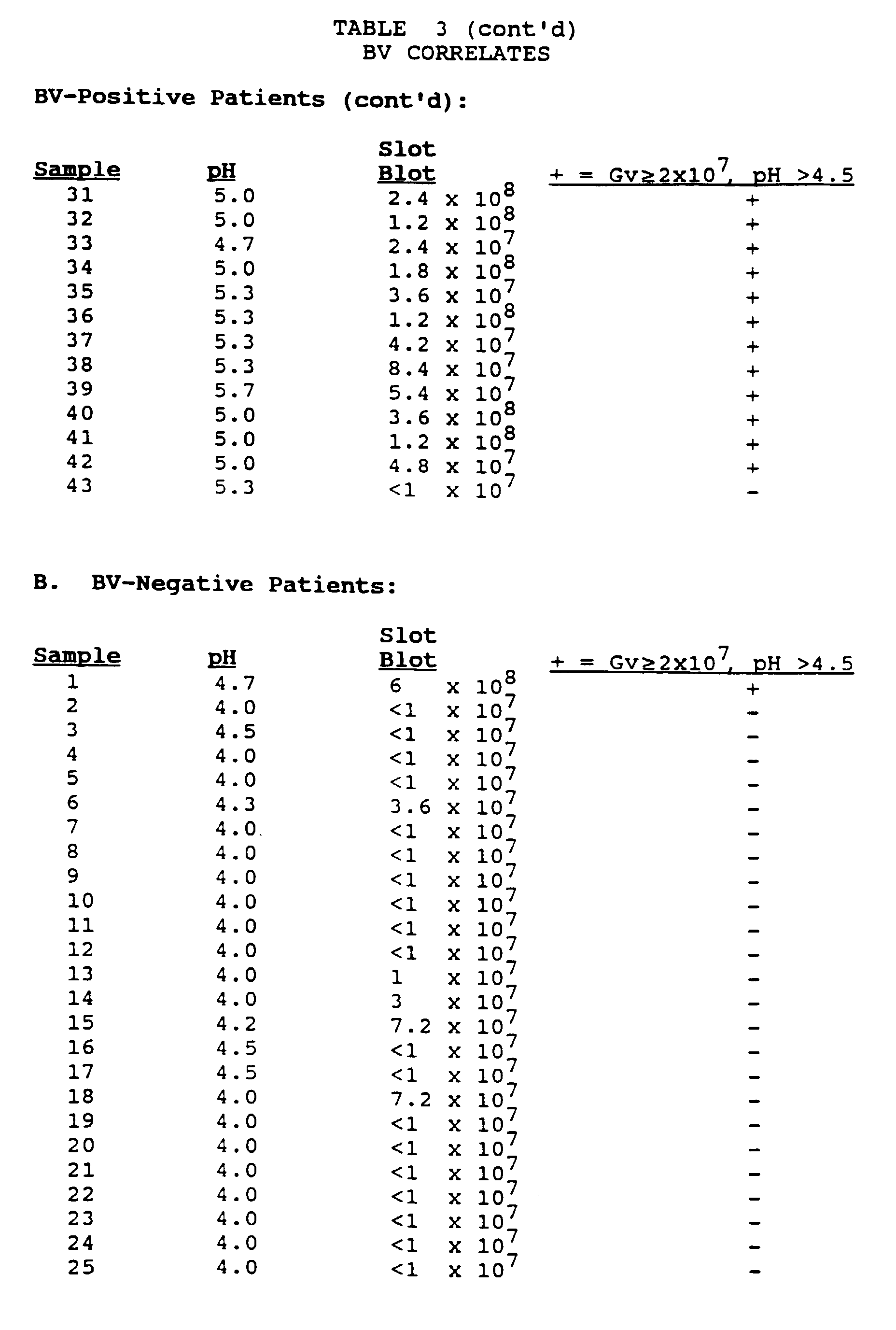

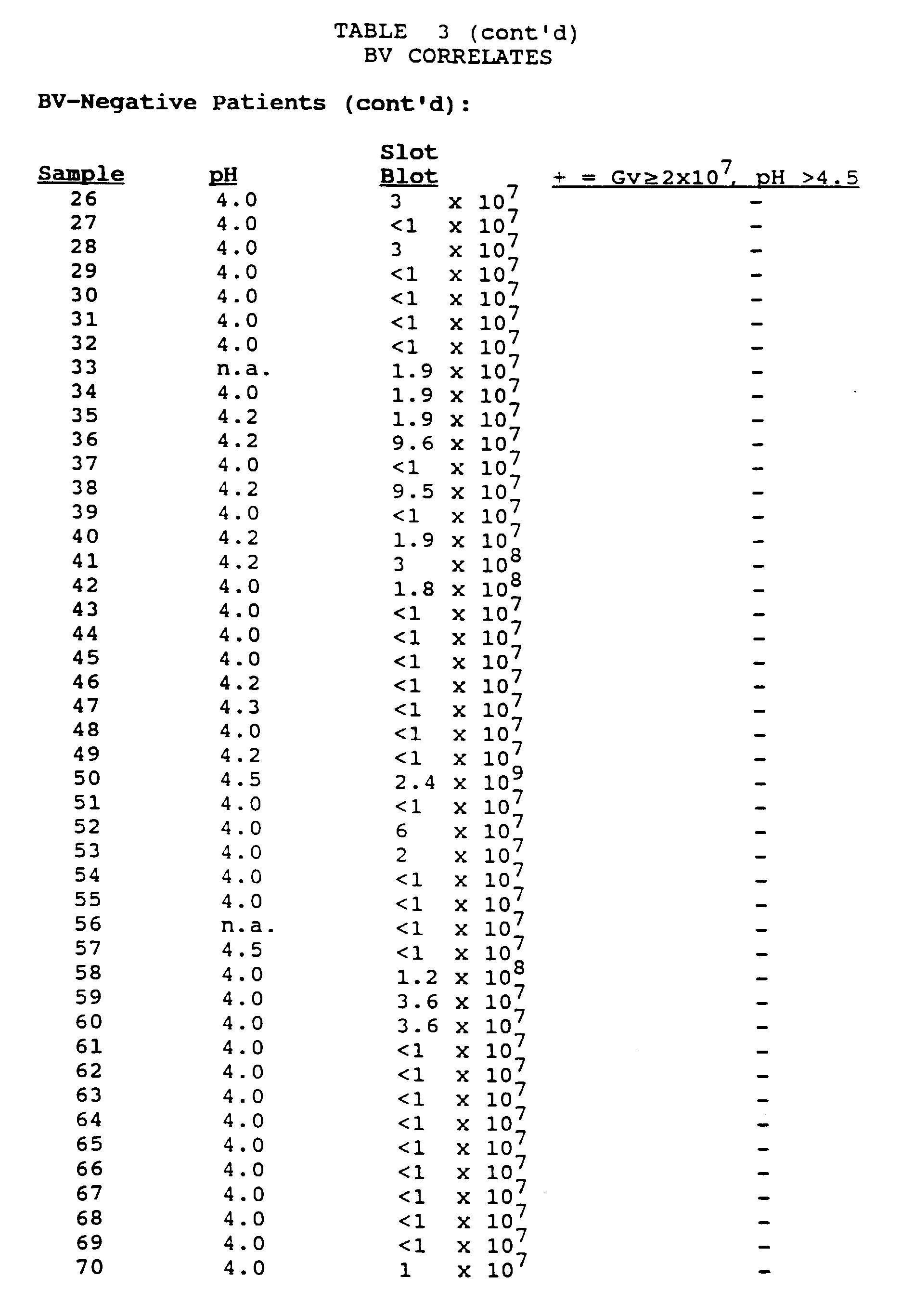

The lower limit of detection for this slot blot analysis was approximately 1 x 10⁷ G. vaginalis cells, based on the signals obtained with the standards. The results showed a strong correlation between the clinical diagnosis of BV and a concentration of G. vaginalis cells greater than or equal to 2 x 10⁷ cells/ml vaginal fluid in women with a vaginal pH greater than 4.5. Using the diagnostic criteria of the present invention, BV was detected with a sensitivity of 95.3% and a specificity of 98.6%. Women with trichomoniasis or cervicitis would be expected to exhibit a vaginal pH >4.5, but G. vaginalis cell numbers < 2 x 10⁷ (i.e., would be diagnosed as BV-negative).

### EXAMPLE 6

Example 6 describes simultaneous detection of Gardnerella vaginalis, Candida spp. and Trichomonas vaginalis in a complex biological sample, and in particular from a vaginal sample.

### Materials:

Hybridization I Solution is 83 mM Tris-Cl, pH 7.5; 17 mM EDTA; 8.35% (v/v) formamide; 5 M GuSCN; and UP041-polymer, 1.67 µg/ml.

Lysis Solution is 90 mM Tris-Cl, pH 8.0; 10 mM EDTA; 5% (w/v) N-lauroylsarcosine; 0.5% (w/v) SDS; and 0.1% Proclin® (preservative).

Substrate Solution B is 1% (w/v) 4-methoxy naphthol; 0.1% (v/v) acetic acid; and isopropanol.

Hybridization II Solution is 3M GuSCN; 0.3M imidazole; 5% (v/v) formamide; 50 mM Tris-Cl, pH 8.5; 10 mM EDTA; and biotinylated probes capable of hybridizing with Gardnerella vaginalis, Candida spp. and Trichomonas vaginalis (UP541 [complement of UP041], 1000 µg/ml, Gv; procedural control probe, 1 µg/ml; UP053, 500 µg/ml, Ca; TRV017, 500 µg/ml, Tv).

Assay Wash Solution is 9.1 mM Tris-Cl, pH 8.0; Na-EDTA; 1% (w/v) N-lauroylsarcosine; 1% (w/v) SDS; and 0.1% (w/v) ProClin® (preservative).

Conjugate Solution is streptavidin-horse radish peroxidase conjugate, 1-3 µg SA/ml.

Substrate Solution I is 100 mM Na-H₂PO₄, pH 6.5; 12 mM citrate; and 1.8% (v/v) hydrogen peroxide.

Oligonucleotide Probes:

CAL015: 5'TTG-TTC-CTC-GTT-AAG-GTA-TTT-ACA-TTG-TAC-TC3'
TRV015: 5'ATC-CTG-AAA-GAC-CCG-AAG-CCT-GTC3'
TRV017: 5'GTC-ATA-AAA-AAC-ATC-TGG-TCC-TGG-TAA-G3'
UP053: 5'GGA-ATT-ACC-GCG-GCT-GCT-GGC3'

### Procedure:

Patient samples are collected from symptomatic females presenting with vaginal complaint. The patients preferably have not been treated with anti-bacterial or anti-fungal medication within the week prior to sample collection, and preferably have not douched within 24 hours of sample collection.

A sterile dacron swab with a pre-scored handle was used to obtain vaginal fluid samples by twisting or rolling the swab against the vaginal wall two or three times, ensuring that the entire circumference of the swab touched the vaginal wall. The swab was then placed in a sample collection tube, the pre-scored handle of the swab was broken, and the tube was capped. The unlubricated speculum was removed from the patient, and the vaginal pH was determined by touching a pH indicator strip to the speculum.

The swab/sample was either: (1) transported at room temperature for immediate processing and analysis; (2) held at 0°C to 8°C for four hours prior to processing and analysis; or (3) held at room temperature for one hour prior to processing and analysis. To initiate processing, 0.3 ml of Lysis Solution was added to the swab/sample, which was then swirled or agitated in the Lysis Solution for about 10-15 seconds. The tube containing the swab and Lysis Solution was heated at 85°C for about 5 minutes (4-8 minutes), 0.45 ml Hybridization I Solution was added to the sample, and the contents of the tube were mixed by flicking about 10 times. At this point, samples may be stored for up to 24 hours at room temperature.

The swab contents were expressed by twirling the swab against the side of the tube. The solution remaining in the tube was then processed on an semi-automated instrument. The sample solution was filtered into the first well of a 7-well reagent cassette. A dipstick containing five beads (i.e, a procedural control, a negative control, Gardnerella vaginalis-specific capture probe GV009, Candida spp.-specific capture probe CAL015, and Trichomonas vaginalis-specific capture probe TRV015) was inserted into Well 1. Substrate Solution B (0.06 ml) was added to Well 7 of the reagent cassette. The arm of the semi-automated instrument clasped the dipstick, and the dipstick was moved from the first well (sample lysate solution) through the following wells: Well 2 = Hybridization II Solution; Well 3 = Assay Wash Solution; Well 4 = Conjugate Solution; Wells 5 and 6 = Assay Wash Solution; and Well 7 = Substrate Solution I. The presence of blue color on a test bead at the end of automated sample processing was indicative of a detectable level of nucleic acid from that target organism. For assay validity, the procedural control bead must turn blue and the negative control bead must remain colorless. The intensity of the blue color on a bead was estimated using an image analyzer.

## Claims

1. A method for simultaneously detecting Gardnerella vaginalis, Candida spp. and Trichomonas vaginalis in a complex biological sample, comprising:
(a) combining the sample and a lysis solution, thereby forming a lysate;
(b) contacting the lysate with a first hybridization solution and with a dipstick comprising a nonporous solid support having at least three capture oligonucleotide-coated beads attached thereto, namely a first bead that specifically hybridizes with Gardnerella vaginalis nucleic acid, a second bead that specifically hybridizes with Candida spp. nucleic acid, and a third bead that specifically hybridizes with Trichomonas vaginalis nucleic acid and;
(c) contacting the dipstick with a second hybridization solution containing at least two signal oligonucleotides, namely a first signal oligonucleotide that specifically hybridizes with prokaryotic nucleic acid, a second signal oligonucleotide that specifically hyridizes with eukaryotic nucleic acid; and
(d) determining signal associated with the beads, thereby detecting the presence of Gardnerella vaginalis, Candida spp. and/or Trichomonas vaginalis in the sample.

2. A method as claimed in claim 1, characterised in that the second hybridization solution includes at least three signal oligonucleotides, namely a first signal oligonucleotide that specifically hybridizes with Gardnerella vaginalis nucleic acid, a second signal oligonucleotide that specifically hybridizes with Candida spp. nucleic acid, and a third signal oligonucleotide that specifically hybridizes with Trichomonas vaginalis nucleic acid.

3. A method as claimed in claim 1 or 2, further comprising the step of determining the pH of the sample.

4. A method as claimed in claim 1, 2 or 3, further comprising heating the lysate at about 85°C for about 5 minutes in the presence of guandinium thiocyanate.

5. A method as claimed in any preceding claim, characterised in that the capture oligonucleotides are complementary to target ribosomal RNA.

6. A method as claimed in any preceding claim, characterised in that the signal oligonucleotide is bound to or is capable of binding to a detectable marker.

7. A method as claimed in claim 6, characterised in that the signal oligonucleotide is bound to biotin and the detectable marker is bound to avidin.

8. A kit for simultaneously detecting Gardnerella vaginalis, Candida spp. and Trichomonas vaginalis, comprising:
(a) a dipstick comprising a nonporous solid support having at least three capture oligonucleotide-coated beads attached thereto, namely a first bead that specifically hybridizes with Gardnerella vaginalis nucleic acid, a second bead that specifically hybridizes with Candida spp. nucleic acid, and a third bead that specifically hybridizes with Trichomonas vaginalis nucleic acid; and
(b) a container including at least two signal oligonucleotides, namely a first signal oligonucleotide that specifically hybridizes with prokaryotic nucleic acid and a second signal oligonucleotide that specifically hybridizes with eukaryotic nucleic acid.

9. A kit as claimed in claim 8, characterised in that the container includes at least three signal oligonucleotides, namely a first signal oligonucleotide that specifically hybridizes with Gardnerella vaginalis nucleic acid, a second signal oligonucleotide that specifically hybridizes with Candida spp. nucleic acid, and a third signal oligonucleotide that specifically hybridizes with Trichomonas vaginalis nucleic acid.

10. A kit as claimed in claim 8 or 9, further comprising a reagent cassette or multi-cavity reagent container.

11. a kit as claimed in claim 8, 9 or 10, further comprising a lysis solution.

## Patentansprüche

1. Verfahren zur simultanen Bestimmung von Gardnerella vaginalis, Candida spp. und Trichomonas vaginalis in einer komplexen biologischen Probe, umfassend:
a) das Kombinieren einer Probe und einer Auflöselösung zur Bildung eines Lysats;
b) das Inkontaktbringen des Lysats mit einer ersten Hybridisierungslösung und mit einem Tauchstab umfassend einen nicht porösen festen Träger, der daran gebunden wenigstens drei mit Einfangoligonukleotid beschichtete Kugeln aufweist, insbesondere eine erste Kugel, die specifisch mit der Nukleinsäure von Gardnerella vaginalis hybridisiert, eine zweite Kugel, die spezifisch mit der Nukleinsäure von Candida spp. hybridisiert und eine dritte Kugel, die spezifisch mit der Nukleinsäure von Trichomonas vaginalis hybridisiert und;
c) das Inkontaktbringen des Tauchstabe mit einer zweiten Hybridisierungslösung, die wenigstens zwei Signaloligonukleotide enthält, nämlich ein erstes Signaloligonukleotid, das spezifisch mit prokaryotischer Nukleinsäure hybridisiert, ein zweites Signaloligonukleotid, das spezifisch mit eukaryotischer Nukleinsäure hybridisiert; und
d) das Bestimmen des mit den Kugeln verbundenen Signals, wobei die Anwesenheit von Gardnerella vaginalis, candida spp. und/oder Trichomonas vaginalis in der Probe bestimmt wird.

2. Verfahren gemäß Anspruch 1, dadurch gekennzeichnet, daß die zweite Hybridisierungslösung wenigstens drei Signaloligonukleotide einschließt, nämlich ein erstes Signaloligonukleotid, das spezifisch mit Nukleinsäure von Gardnerella vaginalis hybridisiert, ein zweites Signaloligonukleotid, das spezifisch mit Nukleinsäure von Candida spp. hybridisiert und ein drittes Signaloligonukleotid, das spezifisch mit Nukleinsäure von Trichomonas vaginalis hybridisiert.

3. Verfahren gemäß Anspruch 1 oder 2, weiter umfassend den Schritt der Bestimmung des pH-Werts der Probe.

4. Verfahren gemäß Anspruch 1, 2 oder 3, weiter umfassend das Erhitzen des Lysats auf etwa 85°C für etwa 5 Minuten in der Gegenwart von Guanidinium Thiocyanat.

5. Verfahren gemäß einem der vorhergehenden Ansprüche, dadurch gekennzeichnet, daß die Einfangoligonukleotide zu der ribosomalen Ziel-RNA komplementär sind.

6. Verfahren nach einem der vorhergehenden Ansprüche, dadurch gekennzeichnet, daß das Signaloligonukleotid an einen bestimmbaren Markierer gebunden ist oder fähig ist, daran zu binden.

7. Verfahren gemäß Anspruch 6, dadurch gekennzeichnet, daß das Signaloligonukleotid an Biotin gebunden ist und der bestimmbare Markierer an Avidin gebunden ist.

8. Kit für die simultane Bestimmung von Gardnerella vaginalis, Candida spp. und Trichomonas vaginalis, umfassend:
a) einen Tauchstab umfassend einen nicht porösen festen Träger der wenigstens drei daran gebundene einfangoligonukleotidbeschichtete Kugeln aufweist, nämlich eine erste Kugel, die spezifisch mit Nukleinsäure von Gardnerella vaginalis hybridisiert, eine zweite Kugel, die spezifisch mit Nukleinsäure von Candida spp. hybridisiert und eine dritte Kugel, die spezifisch mit Nukleinsäure von Trichomonas vaginalis hybridisiert; und
b) ein Behältnis umfassend wenigstens zwei Signaloligonukleotide, nämlich ein erstes Signaloligonukleotid, das spezifisch mit prokaryotischer Nukleinsäure hybridisiert und ein zweites Signaloligonukleotid, das spezifisch mit eukaryotischer Nukleinsäure hybridisiert.

9. Kit nach Anspruch 8, dadurch gekennzeichnet, daß das Behältnis wenigstens drei Signaloligonukleotide umfaßt, nämlich ein erstes Signaloligonukleotid, das spezifisch mit Nukleinsäure von Gardnerella vaginalis hybridisiert, ein zweites Signaloligonukleotid, das spezifisch mit Nukleinsäure von Candida spp. hybridisiert und ein drittes Signaloligonukleotid, das spezifisch mit Nukleinsäure von Trichomonas vaginalis hybridisiert.

10. Ein Kit gemäß Anspruch 8 oder 9, weiterhin umfassend eine Reagenzkassette oder ein Reagenzbehältnis mit vielen Vertiefungen.

11. Ein Kit gemäß Anspruch 8, 9 oder 10, weiter umfassend eine Auflöselösung.

## Revendications

1. Méthode de détection simultanée de *Gardnerella vaginalis,* de *Candida spp.* et de *Trichomonas vaginalis* dans un échantillon biologique complexe, comprenant :
(a) la combinaison de l'échantillon et d'une solution de lyse, formant ainsi un lysat ;
(b) la mise en contact du lysat avec une première solution d'hybridation et avec une bandelette réactive comprenant un support solide non poreux ayant au moins trois billes enrobées d'un oligonucléotide de capture fixées à lui, à savoir une première bille qui s'hybride de manière spécifique avec l'acide nucléique de *Gardnerella vaginalis,* une seconde bille qui s'hybride de manière spécifique avec l'acide nucléique de *Candida spp.* et une troisième bille qui s'hybride de manière spécifique avec l'acide nucléique de *Trichomonas vaginalis* ; et
(c) la mise en contact de la bandelette réactive avec une seconde solution d'hybridation contenant au moins deux oligonucléotides-signaux, à savoir un premier oligonucléotide-signal qui s'hybride de manière spécifique avec l'acide nucléique procaryotique, un second oligonucléotide-signal qui s'hybride de manière spécifique avec l'acide nucléique eucaryotique ; et
(d) la détermination du signal associé aux billes, détectant ainsi la présence de *Gardnerella vaginalis,* de *Candida spp.* et/ou de *Trichomonas vaginalis* dans l'échantillon.

2. Méthode selon la Revendication 1, ***caractérisée en ce que*** la seconde solution d'hybridation comprend au moins trois oligonucléotides-signaux, à savoir un premier oligonucléotide-signal qui s'hybride de manière spécifique avec l'acide nucléique de *Gardnerella vaginalis,* un second oligonucléotide-signal qui s'hybride de manière spécifique avec l'acide nucléique de *Candida spp.* et un troisième oligonucléotide-signal qui s'hybride de manière spécifique avec l'acide nucléique de *Trichomonas vaginalis.*

3. Méthode selon la Revendication 1 ou 2, comprenant additionnellement l'étape de détermination du pH de l'échantillon.

4. Méthode selon la Revendication 1, 2 ou 3, comprenant additionnellement le chauffage du lysat à 85°C environ pendant 5 minutes environ, en présence de thiocyanate de guanidine.

5. Méthode selon l'une quelconque des Revendications précédentes, ***caractérisée en ce que*** les oligonucléotides de capture sont complémentaires d'ARN ribosomique cible.

6. Méthode selon l'une quelconque des Revendications précédentes, ***caractérisée en ce que*** l'oligonucléotide-signal est lié à, ou apte à se lier à, un marqueur détectable.

7. Méthode selon la Revendication 6, ***caractérisée en ce que*** l'oligonucléotide-signal est lié à la biotine et le marqueur détectable est lié à l'avidine.

8. Kit pour la détection simultanée de *Gardnerella vaginalis,* de *Candida spp.* et de *Trichomonas vaginalis,* comprenant :
(a) une bandelette réactive comprenant un support solide non poreux ayant au moins trois billes enrobées d'un oligonucléotide de capture fixées à lui, à savoir une première bille qui s'hybride de manière spécifique avec l'acide nucléique de *Gardnerella vaginalis,* une seconde bille qui s'hybride de manière spécifique avec l'acide nucléique de *Candida spp.* et une troisième bille qui s'hybride de manière spécifique avec l'acide nucléique de *Trichomonas vaginalis* ; et
(b) un récipient englobant au moins deux oligonucléotides-signaux, à savoir un premier oligonucléotide-signal qui s'hybride de manière spécifique avec l'acide nucléique procaryotique, un second oligonucléotide-signal qui s'hybride de manière spécifique avec l'acide nucléique eucaryotique.

9. Kit selon la Revendication 8, ***caractérisé en ce que*** le récipient englobe au moins trois oligonucléotides-signaux, à savoir un premier oligonucléotide-signal qui s'hybride de manière spécifique avec l'acide nucléique de *Gardnerella vaginalis,* un second oligonucléotide-signal qui s'hybride de manière spécifique avec l'acide nucléique de *Candida spp.* et un troisième oligonucléotide-signal qui s'hybride de manière spécifique avec l'acide nucléique de *Trichomonas vaginalis.*

10. Kit selon la Revendication 8 ou 9, comprenant additionnellement une cassette de réactif ou un récipient pour réactif à plusieurs cavités.

11. Kit selon la Revendication 8, 9 ou 10, comprenant additionnellement une solution de lyse.
